# EUROPEAN PATENT APPLICATION

(11) **EP 4 592 271 A1**
(43) Date of publication of application: **30.07.2025**
(21) Application number: 23867474.1
(22) Date of filing: 19.09.2023
(51) Int. Cl.: C07C 39/373, C07C 39/21, C07C 37/055, A61K 31/05, A61P 35/00, A61P 37/00, A61P 17/00

(54) **STILBENE DERIVATIVES AS WELL AS PREPARATION METHOD THEREFOR AND USE THEREOF**

(30) Priority: 22.09.2022 CN 202211170147; 02.06.2023 CN 202310654162
(71) Applicant: Thederma Shanghai Co., Ltd., Shanghai 201203 (CN)
(72) Inventor: CAI, Yaxian, Shanghai 201203 (CN); JIA, Jianmin, Shanghai 201203 (CN); HAN, Zixing, Shanghai 201203 (CN); CAI, Kaiming, Shanghai 201203 (CN); SUN, Huanliang, Shanghai 201203 (CN); TANG, Mengjie, Shanghai 201203 (CN)
(74) Representative: Gille Hrabal Partnerschaftsgesellschaft mbB Patentanwälte
(86) International application number: PCT/CN2023/119575
(87) International publication number: WO 2024/061187

(57) **Abstract**

Provided in the present disclosure are stilbene derivatives, a preparation method therefor and the use thereof. Specifically, provided in the present disclosure is the use of compounds represented by formula (I-1), and stereoisomers, pharmaceutically acceptable salts or prodrugs thereof as an aryl hydrocarbon receptor (AHR) regulator. Compared with marketed drug Benvitimod, the compounds represented by formula I-1 have greatly improved molecular structure stability under illumination, thus overcoming photoinstability of Benvitimod, and solving the problem of Benvitimod being liable to degrade under illumination. In addition, the compounds represented by formula I-1 have remarkably improved activity on AHR protein. And finally, the preparation method for the compounds represented by formula I-1 is simple, and can achieve gram-level or kilogram-level preparation. Therefore, the compounds have better prospects in subsequent preparation development, safety and clinical use.

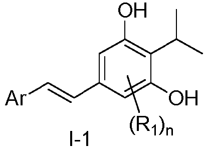

## Description

The present application claims priority to the prior application with the patent application No. 202211170147.6 and entitled "STILBENE DERIVATIVES AS WELL AS PREPARATION METHOD THEREFOR AND USE THEREOF" filed with China National Intellectual Property Administration on September 22, 2022, and the prior application with the patent application No. 202310654162.6 and entitled "STILBENE DERIVATIVES AS WELL AS PREPARATION METHOD THEREFOR AND USE THEREOF" filed with China National Intellectual Property Administration on June 2, 2023, which are incorporated herein by reference in their entirety.

### TECHNICAL FIELD

The present disclosure relates to the technical field of inflammation- or immunity-related medicaments, and particularly, to a novel stilbene derivative, a preparation method therefor, a pharmaceutical composition comprising the derivative, and use of the stilbene derivative and the pharmaceutical composition as a therapeutic agent, especially as an aryl hydrocarbon receptor (AHR) regulator.

### BACKGROUND

The aryl hydrocarbon receptor AHR, also known as the dioxin receptor, is a member of the transcriptional regulator bHLH (basic Helix-Loop-Helix)-PAS (Per-ARNT-Sim) family. The unique characteristic of the members of the bHLH-PAS family is the presence of a PAS domain, named after three proteins in which this motif was first found: Drosophila Per, Human ARNT, and Drosophila Sim. The PAS domain consists of 260-310 amino acids and comprises two highly conserved hydrophobic repeats, known as PAS-A and PAS-B, which are separated by a less conserved sequence. The bHLH domain is responsible for DNA binding, while the tandem PAS domains (PAS-A and PAS-B) are involved in protein-protein interactions and ligand binding. In AHR, ligand binding occurs in the PAS-B domain. The N-terminal bHLH-PAS region in the members of the bHLH-PAS family is relatively well conserved. Most of the non-conservative changes in AHR occur in the transcriptional activation domain, which results in different protein-protein interactions with other co-activators, co-repressors, or nuclear receptors, thereby regulating different gene expressions.

In the absence of ligands, AHR is present in the cytosol and binds to a variety of chaperones, including a dimer of heat shock protein 90 (HSP90), the co-chaperone p23, AHR-interacting protein (AIP), and the protein kinase Src. Upon ligand binding, AHR undergoes a conformational change, translocates into the cell nucleus, dissociates from the chaperone complex, and forms a heterodimer with the aryl hydrocarbon receptor nucleus translocator (ARNT). The regulatory region upstream of the AHR regulatory gene contains a DNA consensus sequence (5'-TNGCGTG-3'), known as the xenobiotic responsive element (XRE) or dioxin responsive element (DRE). This sequence serves as a transcriptional enhancer and is a binding site for AHR. The AHR-ARNT heterodimer complex is recruited by XRE, initiating the transcription of target genes.

Studies have shown that AHR is involved in physiological processes such as cell physiology, host defense, immune cell proliferation and differentiation, detoxification, etc. AHR is expressed in many cells of the immune system, including dendritic cells, macrophages, T cells, NK cells, etc.

Since the ligand-binding site for AHR is structurally flexible, many small molecules can be used as ligands, including exogenous ligands such as polycyclic aromatic hydrocarbons, dioxins, polychlorinated biphenyls, etc., endogenous ligands such as metabolites of tryptophan degradation, food-derived ligands, and products of bacterial and microbial metabolic pathways. For example, Benvitimod, an AHR regulator, is a naturally derived small molecule produced by bacterial symbionts of entomopathogenic nematodes, is the first marketed aryl hydrocarbon receptor agonist in the world, and can be used for treating various autoimmune diseases, such as psoriasis, eczema, etc. However, Benvitimod is easily degraded under illumination due to its own structural characteristics, such as photoinstability, thereby limiting its application. Therefore, the development of an AHR regulator with improved photostability is of great significance for expanding the clinical application range and reducing potential side effects.

### SUMMARY

In order to solve the problems described above in the prior art, the present disclosure provides a compound represented by formula I-1 below and a stereoisomer thereof, a pharmaceutically acceptable salt thereof, or a prodrug thereof: 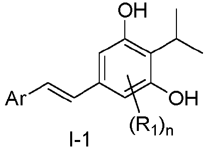
wherein Ar is selected from the following groups unsubstituted or optionally substituted with one, two, or more Rs: C₆₋₂₀ aryl and 5- to 20-membered heteroaryl;
each Rs is identical or different and is independently selected from halogen, cyano, C₁₋₁₂ alkyl, halogenated C₁₋₁₂ alkyl, -COC₁₋₁₂ alkyl, and C₁₋₁₂ alkoxy;
each R₁ is identical or different and is independently selected from halogen, cyano, C₁₋₁₂ alkyl, halogenated C₁₋₁₂ alkyl, -COC₁₋₁₂ alkyl, and C₁₋₁₂ alkoxy; and
n is 1 or 2.

In some embodiments of the present disclosure, provided is the compound represented by formula I-1 and the stereoisomer thereof, the pharmaceutically acceptable salt thereof, or the prodrug thereof, wherein each R₁ is identical or different and is independently selected from halogen, cyano, C₂₋₆ linear alkyl, halogenated C₁₋₆ alkyl, -COC₂₋₆ alkyl, and C₁₋₆ alkoxy.

In some embodiments of the present disclosure, the compound represented by formula I-1 and the stereoisomer thereof, the pharmaceutically acceptable salt thereof, or the prodrug thereof is a compound represented by formula I and a stereoisomer thereof, a pharmaceutically acceptable salt thereof, or a prodrug thereof: 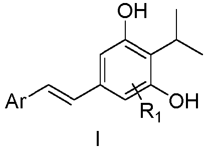 wherein:
Ar is selected from the following groups unsubstituted or optionally substituted with one, two, or more Rs: C₆₋₂₀ aryl and 5- to 20-membered heteroaryl;
each Rs is identical or different and is independently selected from halogen, C₁₋₁₂ alkyl, halogenated C₁₋₁₂ alkyl, -COC₁₋₁₂ alkyl, and C₁₋₁₂ alkoxy;
R₁ is as defined in formula I-1.

Further, the present disclosure provides a compound represented by formula I and a stereoisomer thereof, a pharmaceutically acceptable salt thereof, or a prodrug thereof: 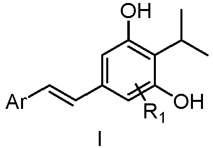
wherein Ar is selected from the following groups unsubstituted or optionally substituted with one, two, or more Rs: C₆₋₂₀ aryl and 5- to 20-membered heteroaryl;
each Rs is identical or different and is independently selected from halogen, C₁₋₁₂ alkyl, halogenated C₁₋₁₂ alkyl, -COC₁₋₁₂ alkyl, and C₁₋₁₂ alkoxy;
R₁ is selected from halogen, cyano, C₁₋₁₂ alkyl, halogenated C₁₋₁₂ alkyl, -COC₁₋₁₂ alkyl, and C₁₋₁₂ alkoxy.

In some embodiments of the present disclosure, provided is the compound represented by formula I-1 or formula I and the stereoisomer thereof, the pharmaceutically acceptable salt thereof, or the prodrug thereof, wherein Ar is selected from the following groups unsubstituted or optionally substituted with one, two, or more Rs: 6- to 10-membered aryl and 5- to 10-membered heteroaryl; Rs is as defined in formula I-1 or formula I; preferably, Ar is selected from the following groups unsubstituted or optionally substituted with one, two, or more Rs: phenyl and 5- to 10-membered heteroaryl; Rs is as defined in formula I-1 or formula I; more preferably, Ar is selected from the following groups unsubstituted or optionally substituted with one, two, or more Rs: phenyl and 5- to 6-membered heteroaryl; Rs is as defined in formula I-1 or formula I; and most preferably, Ar is selected from the following groups unsubstituted or optionally substituted with one, two, or more Rs: phenyl, pyridin-2-yl, pyridin-3-yl, pyridin-4-yl, thienyl, quinolyl, isoquinolyl, pyridazinyl, pyrazinyl, and pyrimidinyl; Rs is as defined in formula I-1 or formula I.

In some embodiments of the present disclosure, provided is the compound represented by formula I-1 or formula I and the stereoisomer thereof, the pharmaceutically acceptable salt thereof, or the prodrug thereof, wherein each R₁ is identical or different and is independently F, Cl, Br, methyl, or cyano.

In some embodiments of the present disclosure, provided is the compound represented by formula I-1 or formula I and the stereoisomer thereof, the pharmaceutically acceptable salt thereof, or the prodrug thereof, wherein each Rs is identical or different and is independently selected from halogen, cyano, C₁₋₆ alkyl, halogenated C₁₋₆ alkyl, -COC₁₋₆ alkyl, and C₁₋₆ alkoxy; preferably, each Rs is identical or different and is independently selected from halogen, cyano, C₁₋₆ alkyl, and C₁₋₆ alkoxy; more preferably, each Rs is identical or different and is independently selected from halogen, C₁₋₆ alkyl, and C₁₋₆ alkoxy; and most preferably, each Rs is identical or different and is independently halogen.

In some embodiments of the present disclosure, provided is the compound represented by formula I-1 or formula I and the stereoisomer thereof, the pharmaceutically acceptable salt thereof, or the prodrug thereof, wherein each Rs is identical or different and is independently selected from halogen, C₁₋₆ alkyl, halogenated C₁₋₆ alkyl, -COC₁₋₆ alkyl, and C₁₋₆ alkoxy; preferably, each Rs is identical or different and is independently selected from halogen, C₁₋₆ alkyl, and C₁₋₆ alkoxy; more preferably, each Rs is identical or different and is independently selected from halogen, C₁₋₆ alkyl, and C₁₋₆ alkoxy; and most preferably, each Rs is identical or different and is independently halogen; for example, Rs is F, Cl, methyl, or methoxy.

In some embodiments of the present disclosure, provided is the compound represented by formula I-1 or formula I and the stereoisomer thereof, the pharmaceutically acceptable salt thereof, or the prodrug thereof, wherein Ar is selected from C₆₋₁₄ aryl and 5- to 14-membered heteroaryl unsubstituted or optionally substituted with one, two, or more of the following groups: halogen, C₁₋₃ alkyl, and C₁₋₃ alkoxy; R₁ is selected from F, Cl, Br, cyano, C₁₋₃ alkyl, halogenated C₁₋₃ alkyl, -COC₁₋₃ alkyl, and C₁₋₃ alkoxy, preferably F, Cl, Br, cyano, C₁₋₃ alkyl, or halogenated C₁₋₃ alkyl.

In some embodiments of the present disclosure, provided is the compound represented by formula I-1 or formula I and the stereoisomer thereof, the pharmaceutically acceptable salt thereof, or the prodrug thereof, wherein Ar is selected from the following groups unsubstituted or optionally substituted with one, two, or more Rs: phenyl, pyridin-2-yl, pyridin-3-yl, pyridin-4-yl, 2-thienyl, 2-pyridazinyl, and 2-quinolyl, and Rs is as defined in formula I-1 or formula I; preferably, Ar is selected from the following groups unsubstituted or optionally substituted with one, two, or more Rs: phenyl, pyridin-2-yl, pyridin-3-yl, pyridin-4-yl, 2-thienyl, 2-pyridazinyl, and 2-quinolyl; Rs is halogen; for example, Ar is selected from phenyl, pyridinyl, thienyl, pyridazinyl, and quinolyl substituted with one, two, or more substituents selected from fluoro, chloro, bromo, methyl, and methoxy, and examples of Ar may be selected from 4-fluorophenyl, 2-fluorophenyl, 2-pyridazinyl, 2-thienyl, 2-quinolyl, 2-pyridinyl, 3-pyridinyl, 4-pyridinyl, 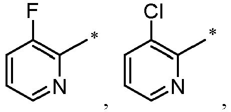 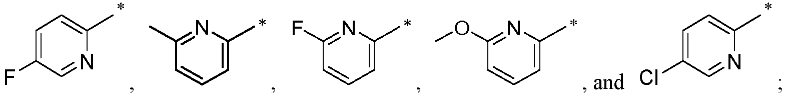 R₁ is selected from F, Cl, Br, methyl, methoxy, cyano, acetyl,

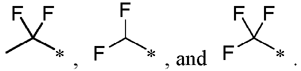

In one embodiment, R₁ is substituted at the ortho position of one of the hydroxyl groups on the phenyl ring, or substituted at the ortho positions of two hydroxyl groups simultaneously.

In one embodiment, when Ar is heteroaryl, it is attached to alkenyl at position 2, position 3, or position 4.

As an example, the compound represented by formula I-1 or formula I is selected from the following, including but not limited to:

**Table A:**

| | | |
|---|---|---|
| 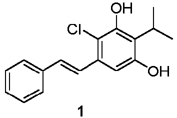 | 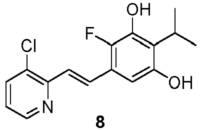 | 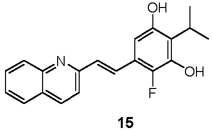 |
| 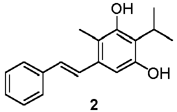 | 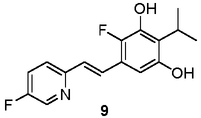 | 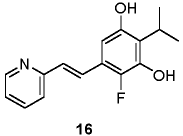 |
| 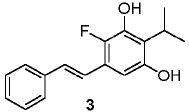 | 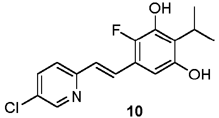 | 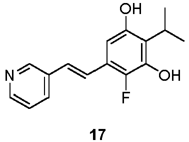 |
| 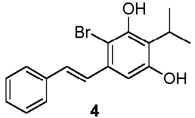 | 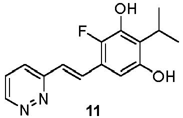 | 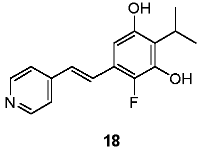 |
| 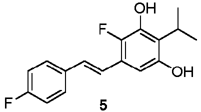 | 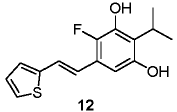 | 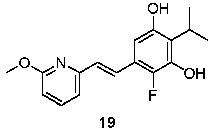 |
| 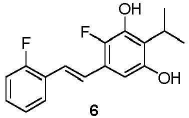 | 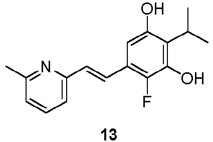 | |
| 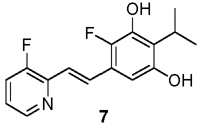 | 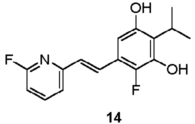 | |

According to an embodiment of the present disclosure, the prodrug may be an ester formed from at least one hydroxyl of the compound represented by formula I-1 or formula I with a pharmaceutically acceptable compound having at least one carboxyl. As an example, the compound having at least one carboxyl may be a monobasic, dibasic, or polybasic organic acid (e.g., acetic acid or phosphoric acid). Or alternatively, when the organic acid is a dibasic or polybasic organic acid, it may be esterified with the compound represented by formula I through 1 carboxyl group, while the other carboxyl groups are reacted with hydroxyl-substituted C₁₋₁₂ alkyl to form an ester.

Another aspect of the present disclosure relates to a compound represented by formula I-1d or a salt thereof: 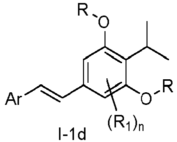 wherein:
R is selected from alkyl, hydroxyalkyl, cycloalkyl, heterocyclyl, aryl, and heteroaryl, wherein the alkyl, cycloalkyl, heterocyclyl, aryl, and heteroaryl are each independently and optionally substituted with one, two, or more substituents selected from the following groups: halogen, oxo (=O), alkyl, haloalkyl, alkoxy, haloalkoxy, nitro, cyano, amino, alkylamino, hydroxyl, hydroxyalkyl, cycloalkyl, heterocyclyl, aryl, and heteroaryl; preferably, R is C₁₋₆ alkyl;
each R₁ is identical or different and is independently selected from halogen, cyano, C₁₋₁₂ alkyl, halogenated C₁₋₁₂ alkyl, -COC₁₋₁₂ alkyl, and C₂₋₁₂ alkoxy, preferably F, Cl, Br, cyano, C₁₋₃ alkyl, or halogenated C₁₋₃ alkyl;
Ar and n are as defined in formula I-1 or formula I.

**Table B. Typical intermediate compounds of the present disclosure include, but are not limited to:**

| | | |
|---|---|---|
| 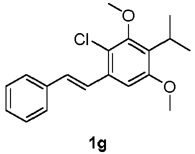 | 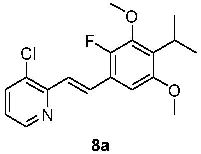 | 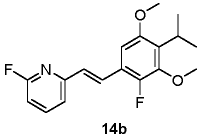 |
| 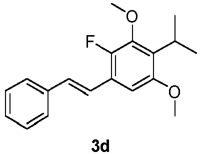 | 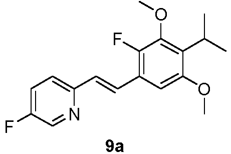 | 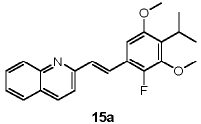 |
| 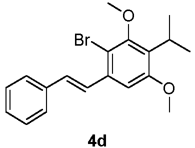 | 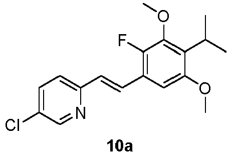 | 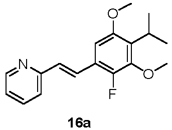 |
| 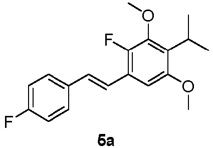 | 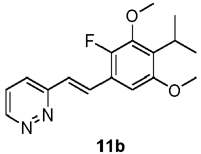 | 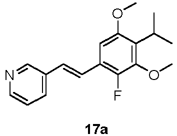 |
| 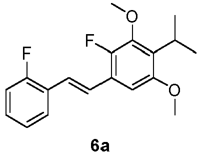 | 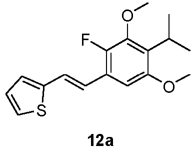 | 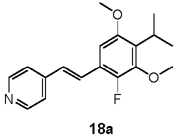 |
| 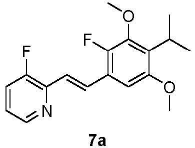 | 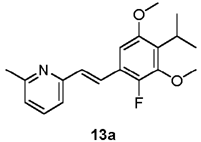 | 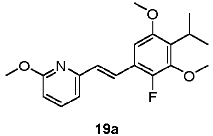 |

Another aspect of the present disclosure relates to a method for preparing a compound represented by formula I-1 and a stereoisomer thereof or a pharmaceutically acceptable salt thereof, the method comprising the following step: 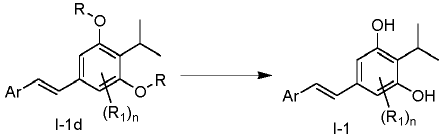
subjecting a compound represented by formula I-1d or a salt thereof to a deprotection reaction under acidic conditions to give the compound represented by formula I-1 and the stereoisomer thereof or the pharmaceutically acceptable salt thereof;
wherein:
   R is selected from alkyl, hydroxyalkyl, cycloalkyl, heterocyclyl, aryl, and heteroaryl, wherein the alkyl, cycloalkyl, heterocyclyl, aryl, and heteroaryl are each independently and optionally substituted with one, two, or more substituents selected from the following groups: halogen, oxo (=O), alkyl, haloalkyl, alkoxy, haloalkoxy, nitro, cyano, amino, alkylamino, hydroxyl, hydroxyalkyl, cycloalkyl, heterocyclyl, aryl, and heteroaryl; preferably, R is C₁₋₆ alkyl;
   each R₁ is identical or different and is independently selected from halogen, cyano, C₂₋₆ linear alkyl, halogenated C₁₋₆ alkyl, -COC₂₋₆ alkyl, and C₁₋₆ alkoxy, preferably F, Cl, Br, cyano, or halogenated C₁₋₃ alkyl;
   Ar and n are as defined in formula I-1 or formula I.

The present disclosure further provides a preparation method for a compound represented by formula I and a stereoisomer thereof or a pharmaceutically acceptable salt thereof, comprising the following steps:
1) when R₁ is halogen, the compound represented by formula I is prepared by the following method: 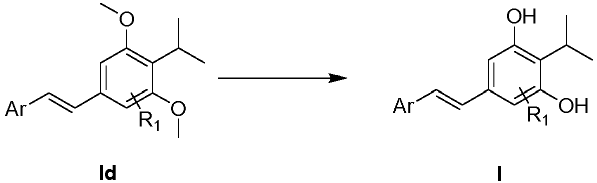 S5) subjecting a compound represented by formula Id or a salt thereof and pyridine hydrochloride to a heating reaction to give the compound represented by formula I; or removing methyl from the compound represented by formula Id or the salt thereof by using boron tribromide, and then quenching the reaction with water to give the compound represented by formula I and the stereoisomer thereof or the pharmaceutically acceptable salt thereof;
2) when R₁ is methyl, the compound represented by formula I is prepared by the following method: 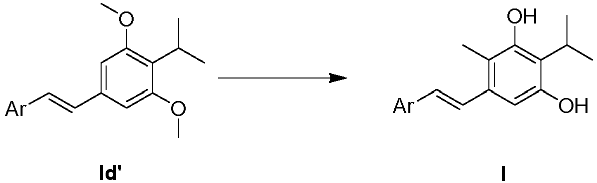
   S5') subjecting compound Id' and pyridine hydrochloride to a heating reaction to give the compound represented by formula I;
   wherein Ar and R₁ are as defined in formula I-1 or formula I.

In one embodiment, compound Id is prepared by the following method, but is not limited thereto: 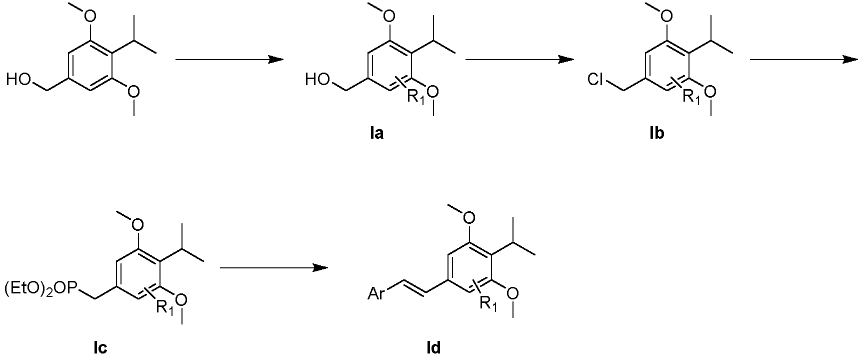
S1) reacting 3,5-dimethoxy-4-isopropylbenzyl alcohol with a halogenation reagent (such as N-chlorosuccinimide, 1-chloromethyl-4-fluoro-1,4-diazabicyclo[2.2.2]octane bis(tetrafluoroborate) or *N*-bromosuccinimide) to give compound Ia;
S2) adding compound Ia to a mixed solution of concentrated hydrochloric acid and n-hexane, and heating for reaction to give compound Ib;
S3) subjecting compound Ib and triethyl phosphite to a heating reaction to give compound Ic; and
S4) reacting compound Ic with compound 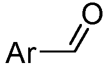 in the presence of an alkaline compound (such as potassium *tert*-butoxide, sodium *tert*-butoxide, and other alkaline compounds) to give compound Id;
the Ar group in the compound 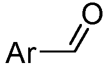 has the same definition as in formula I-1 or formula I above.

Optionally, the preparation method further comprises the step of preparing a salt from the compound represented by formula I-1 or formula I or the compounds shown in Table A.

The present disclosure further provides a pharmaceutical composition comprising a therapeutically effective amount of at least one of the compound represented by formula I-1 or formula I, or the compounds shown in Table A, and the stereoisomer thereof, the pharmaceutically acceptable salt thereof, or the prodrug thereof.

According to an embodiment of the present disclosure, the pharmaceutical composition further comprises one or more pharmaceutically acceptable carriers or excipients.

According to an embodiment of the present disclosure, the pharmaceutical composition is an aryl hydrocarbon receptor (AHR) regulator.

According to an embodiment of the present disclosure, the aryl hydrocarbon receptor (AHR) regulator is used for the alleviation and/or treatment of the following diseases or conditions: a cancer, an ophthalmology-related disease, an autoimmune disease, and other conditions or discomfort with an immunological factor; the cancer is preferably leukemia, prostate cancer, and intestinal cancer; the ophthalmology-related disease is preferably uveitis, age-related macular degeneration, and dry eye syndrome; the autoimmune disease is preferably rheumatoid arthritis, multiple sclerosis, systemic lupus erythematosus, inflammatory bowel disease, type 1 diabetes, vitiligo, atopic dermatitis, and psoriasis; said other conditions or discomfort with the immunological factor are preferably asthma, allergy, infection, osteoporosis, atherosclerosis, type 2 diabetes, graft-versus-host disease, and graft rejection.

The present disclosure further provides use of at least one of the compound represented by formula I-1 or formula I, or the compounds shown in Table A, and the stereoisomer thereof, the pharmaceutically acceptable salt thereof, or the prodrug thereof for manufacturing an aryl hydrocarbon receptor (AHR) regulator.

The present disclosure further provides a method for alleviating and/or treating an aryl hydrocarbon receptor (AHR)-mediated disease or condition, comprising administering to a patient a therapeutically effective amount of at least one of the compound represented by formula I-1 or formula I, or the compounds shown in Table A, and the stereoisomer thereof, the pharmaceutically acceptable salt thereof, or the prodrug thereof, or the pharmaceutical composition as described above.

According to an embodiment of the present disclosure, the aryl hydrocarbon receptor (AHR)-mediated disease or condition comprises: a cancer, an ophthalmology-related disease, an autoimmune disease, and other conditions or discomfort with an immunological factor; the cancer is preferably leukemia, prostate cancer, and intestinal cancer; the ophthalmology-related disease is preferably uveitis, age-related macular degeneration, and dry eye syndrome; the autoimmune disease is preferably rheumatoid arthritis, multiple sclerosis, systemic lupus erythematosus, inflammatory bowel disease, type 1 diabetes, vitiligo, atopic dermatitis, and psoriasis; said other conditions or discomfort with the immunological factor are preferably asthma, allergy, infection, osteoporosis, atherosclerosis, type 2 diabetes, graft-versus-host disease, and graft rejection.

### Beneficial Effects

Compared with the marketed drug Benvitimod, the compounds obtained by improving the structure of Benvitimod have greatly improved molecular structure stability under illumination, thus overcoming photoinstability of Benvitimod, and solving the problem of Benvitimod being easily degraded under illumination. In addition, some compounds have significantly improved activity on AHR protein or achieve an activity that is at least not lower than that of Benvitimod.

Finally, the preparation method for the obtained compounds is simple, and can achieve gram-level or kilogram-level preparation. Therefore, the compounds have better prospects in subsequent formulation development, safety, and clinical use.

### Definitions and Description

Unless otherwise defined, all technical and scientific terms used herein have the same meaning as commonly understood by those skilled in the art to which the subject matter of the claims belong.

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should be construed as being within the scope of the specification and/or the claims of the present application.

Unless otherwise stated, a numerical range set forth in the specification and claims shall be construed as at least including each specific integer value within the range. For example, the numerical range of "1-10" shall be construed as including each integer value in the numerical range "1-10", i.e., 1, 2, 3, 4, 5, 6, 7, 8, 9, and 10. Moreover, when certain numerical ranges are defined as "a number", it shall be construed as including both endpoints of the range, each integer within the range, and each decimal within the range. For example, "a number of 0-10" shall be construed as including not only each of integers 0, 1, 2, 3, 4, 5, 6, 7, 8, 9, and 10, but also at least the sums of each integer and 0.1, 0.2, 0.3, 0.4, 0.5, 0.6, 0.7, 0.8, or 0.9.

It should be understood that when one, two, or more are described herein, "more" shall mean an integer greater than 2, e.g., an integer greater than or equal to 3, e.g., 3, 4, 5, 6, 7, 8, 9, or 10.
"-*" used in combination with a chemical bond in a substituent refers to a connection site.

The term "halogen" includes F, Cl, Br, or I.

The term "alkyl" should be understood to refer to a linear or branched saturated monovalent hydrocarbyl group, such as C₁₋₁₂ alkyl.

The term "C₁₋₁₂ alkyl" should be understood to refer to a linear or branched saturated monovalent hydrocarbyl group having 1-12 carbon atoms, preferably C₁₋₆ alkyl. "C₁₋₆ alkyl" should be understood to preferably refer to a linear or branched saturated monovalent hydrocarbyl group having 1, 2, 3, 4, 5, or 6 carbon atoms, preferably C₂₋₆ alkyl, and more preferably C₂₋₆ linear alkyl. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or an isomer thereof. Particularly, the group has 1, 2, or 3 carbon atoms ("C₁₋₃ alkyl"), such as methyl, ethyl, n-propyl, or isopropyl.

The term "aryl" should be understood to refer to an aromatic or partially aromatic monovalent monocyclic, bicyclic, or tricyclic hydrocarbon ring group having 6 to 20 carbon atoms, preferably C₆₋₂₀ aryl.

The term "C₆₋₂₀ aryl" should be understood to refer to an aromatic or partially aromatic monovalent monocyclic, bicyclic, or tricyclic hydrocarbon ring group having 6 to 20 carbon atoms, preferably "C₆₋₁₄ aryl". The term "C₆₋₁₄ aryl" should be understood to preferably refer to an aromatic or partially aromatic monovalent monocyclic, bicyclic, or tricyclic hydrocarbon ring having 6, 7, 8, 9, 10, 11, 12, 13, or 14 carbon atoms ("C₆₋₁₄ aryl"), preferably 6- to 10-membered aryl, for example, a ring having 6 carbon atoms ("C₆ aryl"), such as phenyl, or biphenyl, or a ring having 9 carbon atoms ("C₉ aryl"), such as indanyl or indenyl, or a ring having 10 carbon atoms ("C₁₀ aryl"), such as tetrahydronaphthyl, dihydronaphthyl, or naphthyl, or a ring having 13 carbon atoms ("C₁₃ aryl"), such as fluorenyl, or a ring having 14 carbon atoms ("C₁₄ aryl"), such as anthryl. When the aryl is substituted, it may be monosubstituted or polysubstituted. In addition, the substitution site is not limited, and may be, for example, ortho-substitution, para-substitution, or meta-substitution.

The term "heteroaryl" should be understood to include a monovalent monocyclic, bicyclic, or tricyclic aromatic ring system that has 5, 6, 7, 8, 9, 10, 11, 12, 13 ... or 20 ring atoms, preferably 5- to 20-membered heteroaryl.

The term "5- to 20-membered heteroaryl" should be understood to include a monovalent monocyclic, bicyclic, or tricyclic aromatic ring system that has 5, 6, 7, 8, 9, 10, 11, 12, 13 ... or 20 ring atoms, particularly 5, 6, 9, or 10 carbon atoms (e.g., preferably 5- to 10-membered heteroaryl), and contains 1-5, preferably 1-3, heteroatoms independently selected from N, O, and S. In addition, the 5- to 20-membered heteroaryl may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl and the like and benzo derivatives thereof, such as benzofuranyl, benzothienyl, benzoxazolyl, benzoisoxazolyl, benzimidazolyl, benzotriazolyl, indazolyl, indolyl, isoindolyl, and the like; or pyridinyl, pyridazinyl, pyrimidinyl, pyrazinyl, triazinyl and the like and benzo derivatives thereof, such as quinolyl, quinazolinyl, isoquinolyl, and the like; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. When the 5- to 10-membered heteroaryl is substituted, it may be monosubstituted or polysubstituted. In addition, the substitution site is not limited. For example, hydrogen attached to the carbon atom on the heteroaryl ring may be substituted, or hydrogen attached to the heteroatom on the heteroaryl ring may be substituted.

Unless otherwise stated, heteroaryl or heteroarylene includes all possible isomeric forms thereof, e.g., position isomers thereof. Thus, for some illustrative non-limiting examples, forms that involving substitutions at or bonding to other groups at one, two, or more of positions 1, 2, 3, 4, 5, 6, 7, 8, 9, 10, and the like (if present) are included, including pyridin-2-yl, pyridinylene-2-yl, pyridin-3-yl, pyridinylene-3-yl, pyridin-4-yl, and pyridinylene-4-yl; thienyl or thienylene, including thien-2-yl, thien-2-ylene, thien-3-yl, and thien-3-ylene; pyrazol-1-yl, pyrazol-3-yl, pyrazol-4-yl, and pyrazol-5-yl.

The term "cycloalkyl" should be understood to refer to a saturated monovalent monocyclic, bicyclic, or polycyclic hydrocarbon ring (also known as fused hydrocarbon ring) having 3-20 carbon atoms, preferably having 3-12 carbon atoms. The bicyclic or polycyclic cycloalkyl includes ortho-fused cycloalkyl, bridged cycloalkyl, and spirocycloalkyl; the ortho-fused ring refers to a fused ring structure formed by two or more ring structures sharing two adjacent ring atoms (i.e., sharing a bond) with each other. The bridged ring refers to a fused ring structure formed by two or more ring structures sharing two non-adjacent ring atoms with each other. The spiro ring refers to a fused ring structure formed by two or more ring structures sharing one ring atom with each other. For example, the cycloalkyl may be C₃₋₈ monocyclic cycloalkyl such as cyclopropyl, cyclobutyl, cyclopentyl, cyclohexyl, cycloheptyl, and cyclooctyl, or may be C₇₋₁₂ ortho-fused cycloalkyl such as a decahydronaphthalene ring.

The term "heterocyclyl" refers to a saturated or partially saturated monovalent monocyclic or bicyclic hydrocarbon ring containing 1-5, preferably 1-3, heteroatoms selected from N, O, and S. The heterocyclyl may be attached to the rest of the molecule through any one of the carbon atoms or a nitrogen atom (if present). In particular, the heterocyclyl may include, but is not limited to, 4- to 20-membered heterocyclyl, e.g., 4-membered rings such as azetidinyl and oxetanyl; 5-membered rings such as tetrahydrofuranyl, dioxolyl, pyrrolidinyl, imidazolidinyl, pyrazolidinyl, and pyrrolinyl; 6-membered rings such as tetrahydropyranyl, piperidyl, morpholinyl, dithianyl, thiomorpholinyl, piperazinyl, and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. The ring containing a nitrogen atom may be partially unsaturated, i.e., it may contain one or more double bonds, for example, but not limited to, 2,5-dihydro-1*H*-pyrrolyl, 4*H*-[1,3,4]thiadiazinyl, 4,5-dihydrooxazolyl, or 4*H*-[1,4]thiazinyl, or it may be benzo-fused, for example, but not limited to, dihydroisoquinolyl. The term "haloalkyl" refers to a group in which H on the alkyl is substituted with halogen, e.g., H on the alkyl is optionally substituted with 1, 2, 3, 4, 5, 6, 7, 8, 9, or 10 halogens, the "halogenated", "alkyl", and "C₁₋₁₂ alkyl" having the definitions as described above. The substitutions are on the same carbon atom, or on different carbon atoms. Optionally "halogenated C₁₋₆ alkyl". The "halogenated C₁₋₁₂ alkyl" is, for example, trifluoromethyl.

The definitions for terms described above are also applicable to other terms containing the term. For example, the definition for the term "C₁₋₁₂ alkyl" described above is also applicable to other terms containing "C₁₋₁₂ alkyl", such as "-COC₁₋₁₂ alkyl", "-COC₂₋₁₂ alkyl", "-COC₂₋₆ alkyl", and the like.

As another example, the term "alkoxy" refers to alkyloxy, wherein the alkyl has the definition as described above. The term "haloalkoxy" refers to haloalkyloxy, wherein the alkyl has the definition as described above.

The term "hydroxyalkyl" refers to hydroxyl-substituted alkyl, wherein the alkyl has the definition as described above. The term "alkylamino" refers to, wherein the alkyl has the definition as described above.

The term "prodrug compound" refers to a covalently-bonded compound that releases the active parent drug according to formula I *in vivo.* Such the prodrug is generally the compound of the present disclosure where one or more appropriate groups have been modified such that, upon administration to a human or mammalian subject, the modification may be reversed. The reversion is typically performed by an enzyme naturally present in such subjects, although it is possible to administer a second medicament with such the prodrug in order to allow for reversion *in vivo.* Examples of such modifications include pharmaceutically acceptable esters as described above, wherein such reversion may be performed via esterases and the like.

### DETAILED DESCRIPTION

The technical solutions of the present disclosure will be further described in detail with reference to the following specific examples. It will be appreciated that the following examples are merely exemplary illustrations and explanations of the present disclosure and should not be construed as limiting the claimed scope of the present disclosure. All techniques implemented on the basis of the content described above of the present disclosure are encompassed within the claimed scope of the present disclosure.

Unless otherwise stated, the starting materials and reagents used in the following examples are all commercially available products or can be prepared by using known methods.

The structures of the compounds in the present disclosure were determined by nuclear magnetic resonance (NMR) or/and mass spectrometry (MS). The NMR shifts (δ) were given in 10⁻⁶ (ppm). NMR analysis was performed on a Bruker AVANCE-400 nuclear magnetic resonance instrument, with deuterated dimethyl sulfoxide (DMSO-*d₆*), deuterated chloroform (CDCl₃), and deuterated methanol (CD₃OD) as solvents and tetramethylsilane (TMS) as an internal standard.

The liquid chromatograph-mass spectrometers used were waters 2695+ZQ2000, Shimadzu MS-2020+LC-20AB, and Shimadzu LC-40D XR+MS-2020.

High performance liquid chromatography (HPLC) analysis was performed on Shimadzu LC-20AB, Shimadzu LC-20ADXR, and Shimadzu LC-40D XR high performance liquid chromatographs.

Chiral HPLC analysis was performed on a Shimadzu LC-30AD high performance liquid chromatograph.

Preparative high performance liquid chromatography was performed on Shimadzu LC-20AP and Gilson GX-281 preparative chromatographs.

If chiral molecules were prepared in the examples, the chiral preparation was performed on Waters 150Mgm and Waters SFC 350 preparative chromatographs.

The CombiFlash preparative flash chromatograph used was CH-200P (Agela & Phenomenex).

Yantai Huanghai HSGF254 or Qingdao GF254 silica gel plate was used as a thin layer chromatography (TLC) silica gel plate. The specification of the silica gel plate for TLC was 0.15-0.2 mm, and that for TLC separation and purification of products was 0.4-0.5 mm.

In the silica gel column chromatography, a 200-300 mesh silica gel (Huanghai, Yantai or Titan Scientific) was generally used as the carrier.

The known starting materials in the present disclosure may be synthesized by using or following methods known in the art, or may be purchased from Titan Scientific, Energy Chemical, Haohong Scientific, Bide Pharmatech, among others. In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen.

In the examples, the reactions were performed under a hydrogen atmosphere, which means that the reaction flask was connected to a balloon containing about 1 L of hydrogen. The pressurized hydrogenation reactions were performed using a Parr 3916EKX hydrogenator and a Qinglan QL-500 hydrogenator, or an HC2-SS hydrogenator. The hydrogenation reactions generally involved 3 cycles of vacuumization and hydrogen purging.

In the examples, the reactions were performed under microwave conditions using a CEM Discover-S 908860 microwave reactor. In the examples, the solutions were aqueous solutions unless otherwise specified.

In the examples, the reactions were performed at room temperature, i.e., 20 °C to 30 °C, unless otherwise specified.

The reaction progress monitoring in the examples was performed using thin layer chromatography (TLC). The developing solvents used in the reactions, the eluent systems used in the column chromatography purification of the compounds, and the developing solvent systems used in the thin layer chromatography include: A: a petroleum ether/ethyl acetate system, and B: a dichloromethane/methanol system. The volume ratio of the solvents was adjusted depending on the polarity of the compounds, or by adding a small amount of basic or acidic reagents such as triethylamine, acetic acid, etc.

In certain embodiments, the purified compounds were prepared by preparative HPLC.

### Example 1

### (E)-4-Chloro-2-isopropyl-5-styrylbenzene-1,3-diol 1

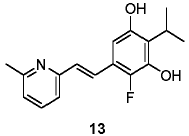

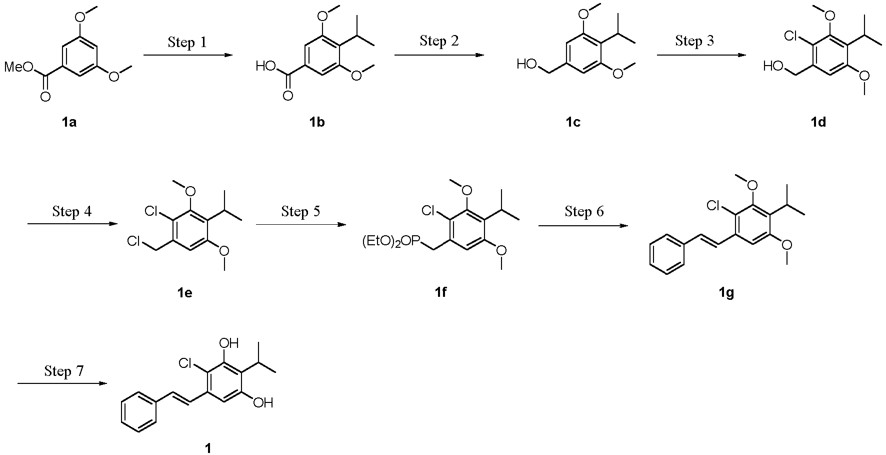

### Step 1

### 4-Isopropyl-3,5-dimethoxybenzoic acid 1b

Water (98 g, 9.44 mol) was carefully added to concentrated sulfuric acid (1.087 kg, 11.007 mol), compound **1a** (250 g, 1.274 mol) was added, and the internal temperature was controlled to be not exceeding 40 °C. Isopropanol (110 g, 1.835 mol) was added dropwise to the mixture described above, and the internal temperature was controlled to be 40-45 °C. After completion of the dropwise addition, the mixture was stirred at 50 °C overnight. The reaction liquid was cooled to room temperature, then slowly poured into ice water (1 kg), and stirred at 40 °C for 1 h. The mixture was filtered, and the filter cake was washed with water and then dissolved in ethyl acetate (250 g). The resulting mixture was heated to reflux for 1 h. The internal temperature was controlled to be 65-70 °C, and n-hexane (1 L) was added. The mixture was stirred for 0.5 h, then cooled to 0 °C, and stirred for another 1 h. The mixture was filtered, and the filter cake was washed with n-hexane and dried overnight at 40 °C to give the title product **1b**.

### Step 2

### (4-Isopropyl-3,5-dimethoxyphenyl)methanol 1c

Compound **1b** (150 g, 0.669 mol) was dissolved in tetrahydrofuran (1 L), and sodium borohydride (39.5 g, 1.037 mol) was added in batches under nitrogen atmosphere. The internal temperature was controlled to be not exceeding 25 °C. Iodine (76.4 g, 0.301 mol) was dissolved in tetrahydrofuran (340 mL) and slowly added dropwise to the mixture described above. The internal temperature was controlled to be 35 °C. After completion of the dropwise addition, the reaction mixture was further stirred at 35 °C overnight. The reaction liquid was cooled to room temperature, poured into water (900 mL), stirred at room temperature for 1 h, and filtered. The filtrate was concentrated to remove the organic solvent, and then added to a sodium bisulfite solution (9 g of sodium bisulfite was dissolved in 900 mL of water). The mixture was stirred at room temperature for 0.5 h and filtered. The filter cake was washed with water and dried overnight at 40 °C to give the title product **1c.**

### Step 3

### (2-Chloro-4-isopropyl-3,5-dimethoxyphenyl)methanol 1d

Compound **1c** (5 g, 23.8 mmol) was dissolved in tetrahydrofuran (50 mL), and a solution ofN-chlorosuccinimide (2.85 g, 21.3 mmol) in tetrahydrofuran (50 mL) was added at 0 °C. The mixture was stirred at room temperature for 2 h. The reaction mixture was diluted with ethyl acetate (100 mL) and washed with water (100 mL) and saturated brine (100 mL). The organic phase was separated out, dried over anhydrous sodium sulfate, and filtered. The filtrate was concentrated, and the residue was purified by silica gel column chromatography to give the title product **1d**.

### Step 4

### 2-Chloro-1-chloromethyl-4-isopropyl-3,5-dimethoxybenzene 1e

Compound **1d** (5.2 g, 21.3 mmol) was added in batches to a mixed solution of concentrated hydrochloric acid (60 mL) and *n*-hexane (50 mL), and the mixture was stirred at 55 °C for 4 h. After cooling to room temperature, the reaction liquid was poured into water (100 mL) and filtered through celite. The organic phase was separated from the filtrate and washed with saturated brine (200 mL), a saturated aqueous sodium bicarbonate solution (200 mL), and water (200 mL). The organic phase was dried over anhydrous sodium sulfate and filtered. The filtrate was concentrated to give the title product **1e.**

### Step 5

### Diethyl (2-chloro-4-isopropyl-3,5-dimethoxybenzyl)phosphate 1f

Compound **1e** (3.2 g, 12.2 mmol) was added to triethyl phosphite (30 mL), and the mixture was heated to 160 °C and stirred for 5 h under nitrogen atmosphere. After cooling to room temperature, the mixture was concentrated to give a crude product of the title product **1f.**

### Step 6

### (E)-2-Chloro-4-isopropyl-3,5-dimethoxy-1-styrylbenzene 1g

Compound **1f** (4.5 g, crude), benzaldehyde (1.2 g, 11.3 mol), and potassium *tert*-butoxide (1.6 g, 14.3 mol) were added to tetrahydrofuran (50 mL), and the mixture was heated to 50 °C and stirred for 2 h under nitrogen atmosphere. After cooling to room temperature, the mixture was diluted with ethyl acetate (50 mL) and washed with water (50 mL) and saturated brine (50 mL). The organic phase was separated out, dried over anhydrous sodium sulfate, and filtered. The filtrate was concentrated, and the residue was purified by silica gel column chromatography to give the title compound 1g.

### Step 7

### (E)-4-Chloro-2-isopropyl-5-styrylbenzene-1,3-diol 1

Compound **1g** (1.3 g, 4.1 mmol) and pyridine hydrochloride (3 g) were mixed, and the mixture was heated to 180 °C and stirred for 3 h under nitrogen atmosphere. After cooling to room temperature, the mixture was diluted with ethyl acetate (50 mL) and washed with water (50 mL) and saturated brine (50 mL). The organic phase was separated out, dried over anhydrous sodium sulfate, and filtered. The filtrate was concentrated, and the residue was purified by silica gel column chromatography to give the title product 1.

LCMS (ESI, m/z): 289.05 [M+H]⁺.

¹H NMR (400 MHz, CDCl₃, ppm): δ 7.54-7.51 (m, 2H), 7.39-7.34 (m, 2H), 7.31-7.25 (m, 2H), 7.01-6.96 (m, 1H), 6.68 (s, 1H), 4.82 (s, 1H), 4.64 (s, 1H), 3.53-3.48 (m, 1H), 1.37 (d, *J* = 6.8 Hz, 6H).

### Example 2

### (E)-2-Isopropyl-4-methyl-5-styrylbenzene-1,3-diol 2

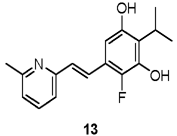

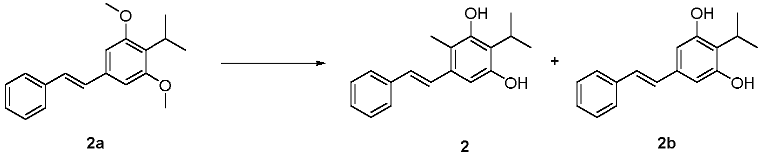

### (E)-2-Isopropyl-4-methyl-5-styrylbenzene-1,3-diol 2

Compound **2a** (3.80 kg) and pyridine hydrochloride (11.15 kg) were added to a 100 L glass reactor under nitrogen atmosphere, and the reaction liquid was heated to 165-175 °C under stirring until the reaction liquid was dissolved, and reacted at a maintained temperature for 5 h. After the reaction was completed, the reaction liquid was cooled to 80-90 °C, poured into diluted hydrochloric acid (obtained by mixing 19.00 kg of water and 0.94 kg of concentrated hydrochloric acid), and stirred. Methyl *tert*-butyl ether (14.05 kg) was added, and the mixture was stirred for 15-20 mins and left to stand for separation. The organic phase was separated out and concentrated under reduced pressure. Methyl *tert*-butyl ether (2.80 kg) was added to the concentrated residue and stirred for complete dissolution. *n*-Heptane (10.40 kg) was added dropwise, and the mixture was placed in an ice-water bath, stirred for 1 h, and filtered. The filtrate was concentrated to give a brown-black oil. The oil described above was purified by silica gel column chromatography and then purified by preparative HPLC to give the title product 2.

LCMS (ESI, m/z): 267.14 [M-H]⁻.

¹H NMR (400 MHz, CDCl₃, ppm): δ 7.38 (d, *J* = 7.4 Hz, 2H), 7.26 (t, *J* = 7.7 Hz, 2H), 7.17-7.15 (m, 2H), 6.75 (d, *J* = 16 Hz, 1H), 6.49 (s, 1H), 4.74 (s, 1H), 4.68 (s, 1H), 3.43-3.34 (m, 1H), 2.13 (s, 3H), 1.30 *(d, J=* 7.2 Hz, 6H).

### Example 3

### (E)-4-Fluoro-2-isopropyl-5-styrylbenzene-1,3-diol 3

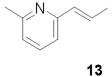

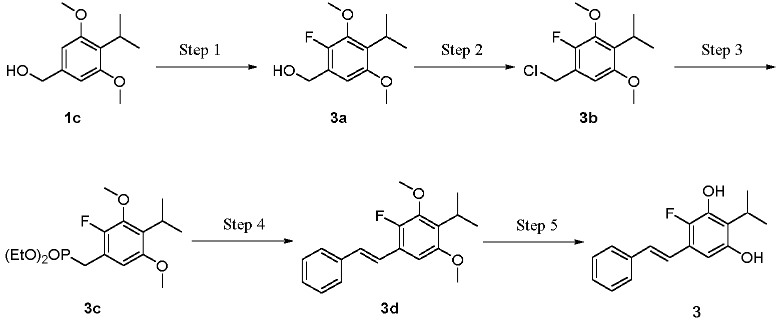

### Step 1

### (2-Fluoro-4-isopropyl-3,5-dimethoxyphenyl)methanol 3a

Compound **1c** (25 g, 118.90 mmol) was dissolved in acetonitrile (250 mL) and placed in an ice-water bath, and 1-chloromethyl-4-fluoro-1,4-diazoniabicyclo[2.2.2]octane bis(tetrafluoroborate) (42.12 g, 118.90 mmol) was added in batches under nitrogen atmosphere. The reaction mixture was stirred at 25 °C for 5 h. The reaction liquid was poured into ethyl acetate (300 mL) and washed with water (300 mL) and saturated brine (300 mL). The organic phase was separated out, dried over anhydrous sodium sulfate, and filtered. The filtrate was concentrated, and the residue was purified by silica gel column chromatography to give the title product **3a**.

### Step 2

### 2-Fluoro-1-chloromethyl-4-isopropyl-3,5-dimethoxybenzene 3b

Referring to the synthesis method of step 4 in Example 1, the title product **3b** was prepared from intermediate **3a**.

### Step 3

### Diethyl (2-fluoro-4-isopropyl-3,5-dimethoxybenzyl)phosphate 3c

Referring to the synthesis method of step 5 in Example 1, the title product **3c** was prepared from intermediate **3b**.

### Step 4

### (E)-2-Fluoro-4-isopropyl-3,5-dimethoxy-1-styrylbenzene 3d

Referring to the synthesis method of step 6 in Example 1, the title product **3d** was prepared from intermediate **3c** and benzaldehyde.

### Step 5

### (E)-4-Fluoro-2-isopropyl-5-styrylbenzene-1,3-diol 3

Referring to the synthesis method of step 7 in Example 1, the title product **3** was prepared from intermediate **3d**. LCMS (ESI, m/z): 273.1 [M+H]⁺.

¹H NMR (400 MHz, CDCl₃, ppm): δ 7.52-7.49 (m, 2H), 7.38-7.34 (m, 2H), 7.29-7.25 (m, 1H), 7.16-7.03 (m, 2H), 6.49 (d, *J=* 6.4 Hz, 1H), 5.22 (d, *J* = 7.2Hz, 1H), 4.64 (s, 1H), 3.50-3.42 (m, 1H), 1.37 (d, *J* = 7.2 Hz, 6H). ¹⁹F NMR (400 MHz, CDCl₃, ppm): δ -154.66 (1F).

### Example 4

### (E)-4-Bromo-2-isopropyl-5-styrylbenzene-1,3-diol 4

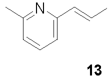

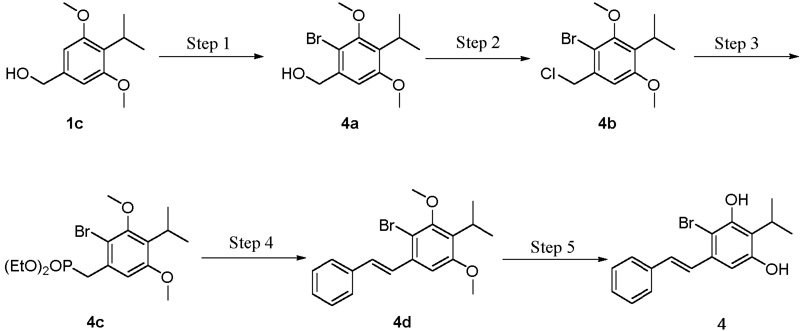

### Step 1

### (2-Bromo-4-isopropyl-3,5-dimethoxyphenyl)methanol 4a

Compound **1c** (10 g, 47.6 mmol) was dissolved in tetrahydrofuran (100 mL), and a solution of *N*-bromosuccinimide (7.62 g, 42.8 mmol) in tetrahydrofuran (30 mL) was added dropwise at room temperature. The mixture was stirred at room temperature for 3 h. The reaction mixture was diluted with ethyl acetate (100 mL) and washed with water (50 mL) and saturated brine (50 mL). The organic phase was separated out, dried over anhydrous sodium sulfate, and filtered. The filtrate was concentrated, and the residue was purified by silica gel column chromatography to give compound **4a**.

### Step 2

### 2-Bromo-1-chloromethyl-4-isopropyl-3,5-dimethoxybenzene 4b

Referring to the synthesis method of step 4 in Example 1, the title product **4b** was prepared from intermediate **4a**.

### Step 3

### Diethyl (2-bromo-4-isopropyl-3,5-dimethoxybenzyl)phosphate 4c

Referring to the synthesis method of step 5 in Example 1, the title product **4c** was prepared from intermediate **4b**.

### Step 4

### (E)-2-Bromo-4-isopropyl-3,5-dimethoxy-1-styrylbenzene 4d

Referring to the synthesis method of step 6 in Example 1, the title product **4d** was prepared from intermediate **4c** and benzaldehyde.

### Step 5

### (E)-4-Bromo-2-isopropyl-5-styrylbenzene-1,3-diol 4

Referring to the synthesis method of step 7 in Example 1, the title product 4 was prepared from intermediate **4d**. LCMS (ESI, m/z): 333.08 [M+H]⁺.

¹H NMR (400 MHz, CDCl₃, ppm): δ 7.54-7.51 (m, 2H), 7.39-7.34 (m, 2H), 7.32-7.25 (m, 2H), 6.97-6.92 (m, 1H), 6.69 (s, 1H), 5.80 (s, 1H), 4.85 (s, 1H), 3.57-3.49 (m, 1H), 1.36 (d, *J* = 7.2 Hz, 6H).

### Example 5

### (E)-4-Fluoro-5-(4-fluorostyryl)-2-isopropylbenzene-1,3-diol 5

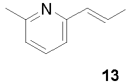

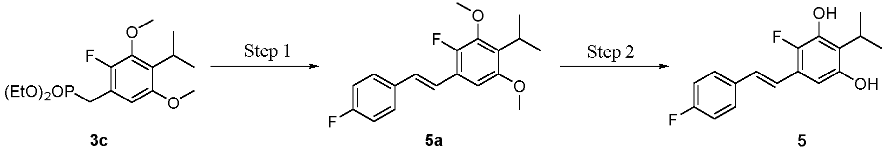

### Step 1

### (E)-2-Fluoro-1-(4-fluorostyryl)-4-isopropyl-3,5-dimethoxybenzene 5a

Referring to the synthesis method of step 6 in Example 1, the title product **5a** was prepared from intermediate **3c** and 4-fluorobenzaldehyde.

### Step 2

### (E)-4-Fluoro-5-(4-fluorostyryl)-2-isopropylbenzene-1,3-diol 5

Referring to the synthesis method of step 7 in Example 1, the title product **5** was prepared from intermediate **5a**. LCMS (ESI, m/z): 291.74 [M+H]⁺.

¹H NMR (400 MHz, DMSO-*d₆*, ppm): δ 9.09 (d, *J* = 2.8 Hz, 1H), 9.05 (s, 1H), 7.67-7.62 (m, 2H), 7.22-7.17 (m, 2H), 7.14-6.99 (m, 2H), 6.51 (d, *J =* 6.0 Hz, 1H), 3.49-3.41 (m, 1H), 1.27 (d, *J* = 7.2 Hz, 6H). ¹⁹F NMR (400 MHz, DMSO-*d₆,* ppm): δ -114.09 (1F), -150.87 (1F).

### Example 6

### (E)-4-Fluoro-5-(2-fluorostyryl)-2-isopropylbenzene-1,3-diol 6

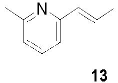

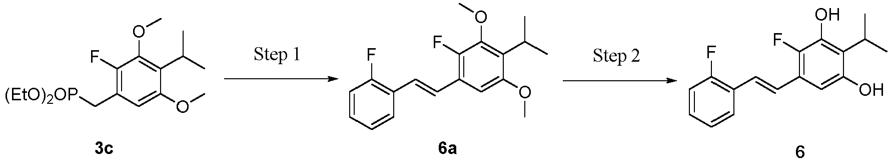

### Step 1

### (E)-2-Fluoro-1-(2-fluorostyryl)-4-isopropyl-3,5-dimethoxybenzene 6a

Referring to the synthesis method of step 6 in Example 1, the title product **6a** was prepared from intermediate **3c** and 2-fluorobenzaldehyde.

### Step 2

### (E)-4-Fluoro-5-(2-fluorostyryl)-2-isopropylbenzene-1,3-diol 6

Referring to the synthesis method of step 7 in Example 1, the title product **6** was prepared from intermediate **6a**. LCMS (ESI, m/z): 291.26 [M+H]⁺.

¹H NMR (400 MHz, DMSO-*d₆*, ppm): δ 9.15 (d, *J* = 2.4 Hz, 1H), 9.09 (s, 1H), 7.83-7.78 (m, 1H), 7.37-7.31 (m, 1H), 7.28-7.21 (m, 3H), 7.14-7.09 (m, 1H), 6.54 (d, *J* = 6.0 Hz, 1H), 3.49-3.41 (m, 1H), 1.25 (d, *J =* 7.2 Hz, 6H); ¹⁹F NMR (400 MHz, DMSO-*d₆*, ppm): δ -118.95 (1F), -150.76 (1F).

### Example 7

### (E)-4-Fluoro-5-[2-(3-fluoropyridin-2-yl)vinyl]-2-isopropylbenzene-1,3-diol 7

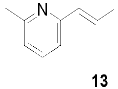

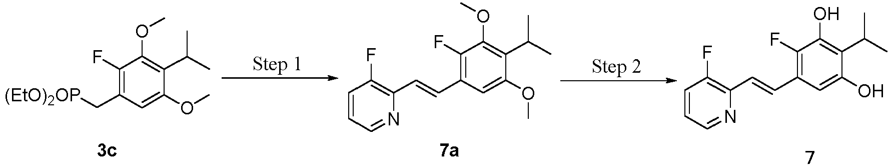

### Step 1

### (E)-3-Fluoro-2-(2-fluoro-4-isopropyl-3,5-dimethoxystyryl)pyridine 7a

Referring to the synthesis method of step 6 in Example 1, the title product **7a** was prepared from intermediate **3c** and 3-fluoro-2-pyridinecarboxaldehyde.

### Step 2

### (E)-4-Fluoro-5-[2-(3-fluoropyridin-2-yl)vinyl]-2-isopropylbenzene-1,3-diol 7

Compound **7a** (100 mg, 0.31 mmol) was dissolved in dichloromethane (10 mL), and boron tribromide (1.3 mL, 1.3 mmol, 1 M solution in dichloromethane) was added dropwise at 0 °C under nitrogen atmosphere. The mixture was stirred at room temperature for 0.5 h. The mixture was quenched with a saturated sodium bicarbonate solution (50 mL) and diluted with dichloromethane (50 mL). The organic phase was separated out, washed with saturated brine (50 mL), dried over anhydrous sodium sulfate, and filtered. The filtrate was concentrated, and the residue was purified by silica gel column chromatography to give compound **7**.

LCMS (ESI, m/z): 292.42 [M+H]⁺.

¹H NMR (400 MHz, DMSO-*d₆*, ppm): δ 9.23 (s, 1H), 9.15 (s, 1H), 8.48-8.45 (m, 1H), 7.79-7.71 (m, 2H), 7.41-7.36 (m, 1H), 7.26-7.19 (m, 1H), 6.60 (d, *J* = 5.2 Hz, 1H), 3.49-3.42 (m, 1H), 1.26 (d, *J* = 6.4 Hz, 6H). ¹⁹F NMR (400 MHz, DMSO-*d₆*, ppm): δ -127.60 (1F), -150.31 (1F).

### Example 8

### (E)-5-[2-(3-Chloropyridin-2-yl)vinyl]-4-fluoro-2-isopropylbenzene-1,3-diol 8

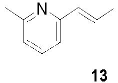

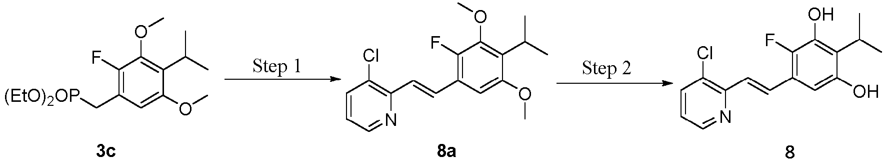

### Step 1

### (E)-3-Chloro-2-(2-fluoro-4-isopropyl-3,5-dimethoxystyryl)pyridine 8a

Referring to the synthesis method of step 6 in Example 1, the title product **8a** was prepared from intermediate **3c** and 3-chloro-2-pyridinecarboxaldehyde.

### Step 2

### (E)-5-[2-(3-Chloropyridin-2-yl)vinyl]-4-fluoro-2-isopropylbenzene-1,3-diol 8

Referring to the synthesis method of step 2 in Example 7, the title product 8 was prepared from intermediate **8a**. LCMS (ESI, m/z): 308.28 [M+H]⁺.

¹H NMR (400 MHz, DMSO-*d₆*, ppm): δ 9.25 (d, *J =* 2.4 Hz, 1H), 9.18 (s, 1H), 8.56 (dd, *J* = 4.4 Hz, 1.2 Hz, 1H), 7.94 (dd, *J* = 8.0 Hz, 1.2 Hz, 1H), 7.84-7.80 (m, 1H), 7.48-7.43 (m, 1H), 7.33 (dd, *J* = 8.0 Hz, 4.4 Hz, 1H), 6.61 (d, *J =* 6.0 Hz, 1H), 3.50-3.42 (m, 1H), 1.26 (d, *J* = 7.2 Hz, 6H). ¹⁹F NMR (400 MHz, DMSO-*d₆*, ppm): δ -149.99 (1F).

### Example 9

### (E)-4-Fluoro-5-[2-(5-fluoropyridin-2-yl)vinyl]-2-isopropylbenzene-1,3-diol 9

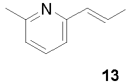

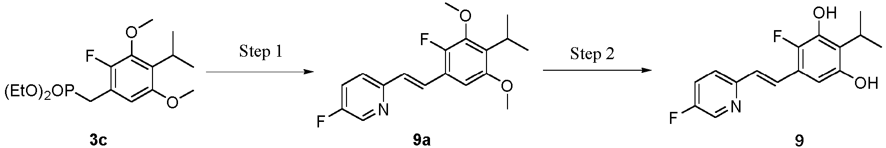

### Step 1

### (E)-5-Fluoro-2-(2-fluoro-4-isopropyl-3,5-dimethoxystyryl)pyridine 9a

Referring to the synthesis method of step 6 in Example 1, the title product **9a** was prepared from intermediate **3c** and 5-fluoro-2-pyridinecarboxaldehyde.

### Step 2

### (E)-4-Fluoro-5-[2-(5-fluoropyridin-2-yl)vinyl]-2-isopropylbenzene-1,3-diol 9

Referring to the synthesis method of step 2 in Example 7, the title product **9** was prepared from intermediate **9a**. LCMS (ESI, m/z): 292.34 [M+H]⁺.

¹H NMR (400 MHz, DMSO-*d₆*, ppm): δ 9.17 (d, *J* = 2.4 Hz, 1H), 9.12 (s, 1H), 8.56 (d, *J* = 2.8 Hz, 1H), 7.75-7.64 (m, 2H), 7.58-7.53 (m, 1H), 7.11-7.06 (m, 1H), 6.54 (d, *J* = 6.0 Hz, 1H), 3.49-3.41 (m, 1H), 1.26 (d, *J* = 7.2 Hz, 6H). ¹⁹F NMR (400 MHz, DMSO-*d₆*, ppm): δ -128.86 (1F), -150.44 (1F).

### Example 10

### (E)-5-[2-(5-Chloropyridin-2-yl)vinyl]-4-fluoro-2-isopropylbenzene-1,3-diol 10

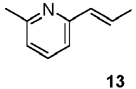

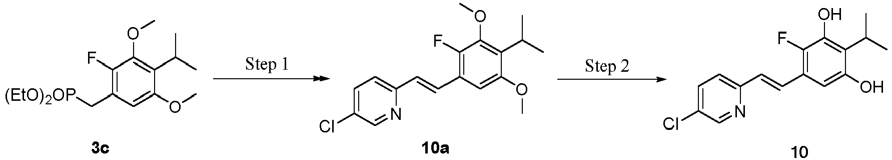

### Step 1

### (E)-5-Chloro-2-(2-fluoro-4-isopropyl-3,5-dimethoxystyryl)pyridine 10a

Referring to the synthesis method of step 6 in Example 1, the title product **10a** was prepared from intermediate **3c** and 5-chloro-2-pyridinecarboxaldehyde.

### Step 2

### (E)-5-[2-(5-Chloropyridin-2-yl)vinyl]-4-fluoro-2-isopropylbenzene-1,3-diol 10

Referring to the synthesis method of step 2 in Example 7, the title product **10** was prepared from intermediate **10a**. LCMS (ESI, m/z): 308.05 [M+H]⁺.

¹H NMR (400 MHz, DMSO-*d₆*, ppm): δ 9.19 (d, *J* = 2.0 Hz, 1H), 9.14 (s, 1H), 8.60 (d, *J* = 2.4 Hz, 1H), 7.91 (dd, *J* = 8.4 Hz, 2.4 Hz, 1H), 7.66-7.60 (m, 2H), 7.10-7.06 (m, 1H), 6.56 (d, *J =* 5.6 Hz, 1H), 3.79-3.39 (m, 1H), 1.25 (d, *J* = 7.2 Hz, 6H). ¹⁹F NMR (400 MHz, DMSO-*d₆*, ppm): δ -150.19 (1F).

### Example 11

### (E)-4-Fluoro-2-isopropyl-5-[2-(pyridazin-3-yl)vinyl]benzene-1,3-diol 11

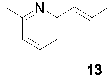

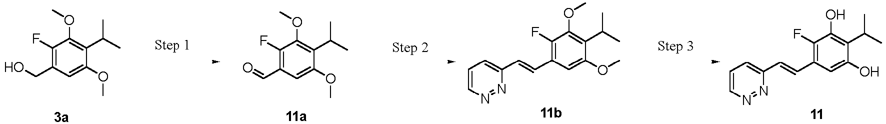

### Step 1

### 2-Fluoro-4-isopropyl-3,5-dimethoxybenzaldehyde 11a

Compound **3a** (15 g, 65.71 mmol) was dissolved in dichloromethane (300 mL), and Dess-Martin periodinane (30.66 g, 72.29 mmol) was added. The mixture was stirred at 20-25 °C for 16 h. A saturated sodium bicarbonate solution (200 mL) was added to the reaction mixture. The organic phase was separated out, washed with saturated brine (100 mL), dried over anhydrous sodium sulfate, and filtered. The filtrate was concentrated, and the residue was purified by silica gel column chromatography to give the title product **11a**.

### Step 2

### (E)-3-(2-Fluoro-4-isopropyl-3,5-dimethoxystyryl)pyridazine 11b

Compound **11a** (240.3 mg, 1.0 mmol) and 3-methylpyridazine (100 mg, 1.0 mmol) were dissolved in 2-methyl-2-butanol (1.5 mL), and potassium hydroxide (59.6 mg, 1.0 mmol) was added. The mixture was heated to 120 °C and stirred for 0.5 h. The reaction was cooled to room temperature, and a saturated aqueous ammonium chloride solution (100 mL) and water (100 mL) were added to the reaction mixture, followed by extraction with ethyl acetate (100 mL). The organic phase was separated out, washed with saturated brine (100 mL), dried over anhydrous sodium sulfate, and filtered. The filtrate was concentrated, and the residue was purified by silica gel column chromatography to give the title product **11b**.

### Step 3

### (E)-4-Fluoro-2-isopropyl-5-[2-(pyridazin-3-yl)vinyl]benzene-1,3-diol 11

Referring to the synthesis method of step 2 in Example 7, the title product **11** was prepared from intermediate **11b**. LCMS (ESI, m/z): 275.1 [M+H]⁺.

¹H NMR (400 MHz, DMSO-*d₆*, ppm): δ 9.29-9.26 (m, 1H), 9.24-9.21 (m, 1H), 9.11-9.08 (m, 1H), 8.03 (dd, *J =* 8.4 Hz, 1.6 Hz, 1H), 7.73 (d, *J =* 16.4 Hz, 1H), 7.68 (dd, *J* = 8.4 Hz, 4.8 Hz, 1H), 7.23 (d, *J =* 16.4 Hz, 1H), 6.60 (d, *J* = 5.6 Hz, 1H), 3.51-3.42 (m, 1H), 1.26 (d, *J* = 7.2 Hz, 6H). ¹⁹F NMR (400 MHz, DMSO-*d₆*, ppm): δ -149.71 (1F).

### Example 12

### (E)-4-Fluoro-2-isopropyl-5-[2-(thien-2-yl)vinyl]benzene-1,3-diol 12

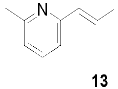

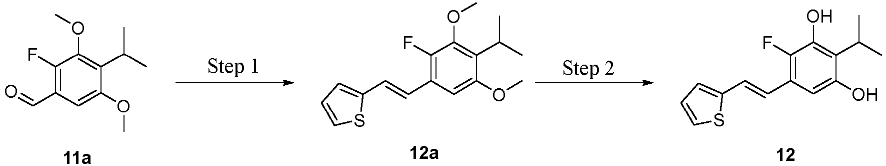

### Step 1

### (E)-2-(2-Fluoro-4-isopropyl-3,5-dimethoxystyryl)thiophene 12a

Referring to the synthesis method of step 6 in Example 1, the title product **12a** was prepared from intermediate **11a** and diethyl (thien-2-ylmethyl)phosphate.

### Step 2

### (E)-4-Fluoro-2-isopropyl-5-[2-(thien-2-yl)vinyl]benzene-1,3-diol 12

Referring to the synthesis method of step 2 in Example 7, the title product **12** was prepared from intermediate **12a**. LCMS (ESI, m/z): 279.0 [M+H]⁺.

¹H NMR (400 MHz, DMSO-*d₆*, ppm): δ 9.14 (s, 1H), 9.11-9.05 (m, 1H), 7.48 (d, *J* = 5.2 Hz, 1H), 7.25 (d, *J =* 3.2 Hz, 1H), 7.19 (d, *J* = 16.4 Hz, 1H), 7.07 (dd, *J* = 5.2 Hz, 3.6 Hz, 1H), 6.83 (d, *J* = 16.4 Hz, 1H), 6.46 (d, *J* = 6.0 Hz, 1H), 3.47-3.40 (m, 1H), 1.24 (d, *J* = 7.2 Hz, 6H). ¹⁹F NMR (400 MHz, DMSO-*d₆*, ppm): δ -151.18 (1F).

### Example 13

### (E)-4-Fluoro-2-isopropyl-5-[2-(6-methylpyridin-2-yl)vinyl]benzene-1,3-diol 13

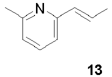

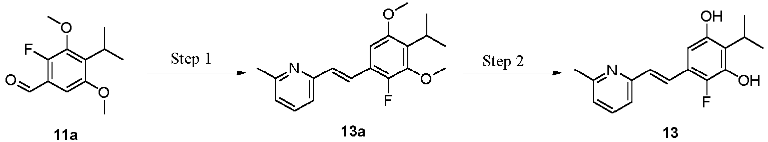

### Step 1

### (E)-2-(2-Fluoro-4-isopropyl-3,5-dimethoxystyryl)-6-methylpyridine 13a

Referring to the synthesis method of step 6 in Example 1, the title product **13a** was prepared from intermediate **11a** and [(6-methylpyridin-2-yl)methyl]triphenylphosphonium bromide.

### Step 2

### (E)-4-Fluoro-2-isopropyl-5-[2-(6-methylpyridin-2-yl)vinyl]benzene-1,3-diol 13

Referring to the synthesis method of step 2 in Example 7, the title product **13** was prepared from intermediate **13a**. LCMS (ESI, m/z): 288.1 [M+H]⁺.

¹H NMR (400 MHz, CDCl₃, ppm): δ 7.64-7.53 (m, 2H), 7.27-7.25 (m, 1H), 7.16 *(d, J=* 16.4 Hz, 1H), 7.05 *(d, J=* 7.6 Hz, 1H), 6.54 (d, *J* = 6.4 Hz, 1H), 3.52-3.45 (m, 1H), 2.60 (s, 3H), 1.38 (d, *J* = 7.2 Hz, 6H). ¹⁹F NMR (400 MHz, CDCl₃, ppm): δ -153.59 (1F).

### Example 14

### (E)-4-Fluoro-5-[2-(6-fluoropyridin-2-yl)vinyl]-2-isopropylbenzene-1,3-diol 14

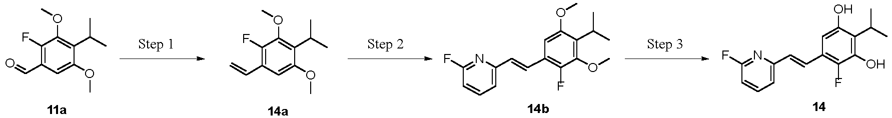

### Step 1

### 2-Fluoro-4-isopropyl-3,5-dimethoxy-1-vinylbenzene 14a

The compound methyltriphenylphosphine bromide (4.7 g, 13.2 mmol) was dissolved in dioxane (30 mL), and potassium carbonate (3.6 g, 26.5 mmol) and **11a** (3 g, 13.2 mmol) were added. The mixture was heated to 110 °C and stirred for 6 h. The system was cooled to room temperature, water (50 mL) was added, and the mixture was extracted with ethyl acetate (50 mL). The organic phase was separated out, washed with saturated brine (50 mL), dried over anhydrous sodium sulfate, and filtered. The filtrate was concentrated, and the residue was purified by silica gel column chromatography to give the title product **14a**.

### Step 2

### (E)-2-Fluoro-6-(2-fluoro-4-isopropyl-3,5-dimethoxystyryl)pyridine 14b

Compound **14a** (1.00 g, 4.46 mmol) and 2-bromo-6-fluoropyridine (1.18 g, 6.69 mmol) were dissolved in dioxane (10 mL). The system was purged three times with nitrogen, and triethylamine (1.35 g, 13.3 mmol) and (1,1'-bis(diphenylphosphino)ferrocene)dichloropalladium(II) (326 mg, 445 µmol) were added. The mixture was heated to 95 °C and stirred for 16 h. The system was cooled to room temperature, water (50 mL) was added, and the mixture was extracted with ethyl acetate (50 mL). The organic phase was separated out, washed with saturated brine (50 mL), dried over anhydrous sodium sulfate, and filtered. The filtrate was concentrated, and the residue was purified by silica gel column chromatography to give the title product **14b**.

### Step 3

### (E)-4-Fluoro-5-[2-(6-fluoropyridin-2-yl)vinyl]-2-isopropylbenzene-1,3-diol 14

Referring to the synthesis method of step 2 in Example 7, the title product **14** was prepared from intermediate **14b**. LCMS (ESI, m/z): 292.1 [M+H]⁺.

¹H NMR (400 MHz, DMSO-*d₆*, ppm): δ 9.23 (d, *J* = 2.4 Hz, 1H), 9.17 (s, 1H), 8.01-7.94 (m, 1H), 7.59 (d, *J* = 16.0 Hz, 1H), 7.50-7.46 (m, 1H), 7.10-7.00 (m, 2H), 6.55 (d, *J* = 6.0 Hz, 1H), 3.49-3.42 (m, 1H), 1.26 (d, *J =* 7.2 Hz, 6H). ¹⁹F NMR (400 MHz, DMSO-*d₆*, ppm): δ -66.80 (1F), -150.07 (1F).

### Example 15

### (E)-4-Fluoro-2-isopropyl-5-[2-(quinolin-2-yl)vinyl]benzene-1,3-diol 15

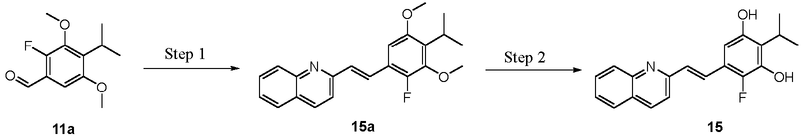

### Step 1

### (E)-2-(2-Fluoro-4-isopropyl-3,5-dimethoxystyryl)quinoline 15a

The compound 2-methylquinoline (300 mg, 2.10 mmol) and **11a** (474 mg, 2.10 mmol) were dissolved in acetic anhydride (5 mL), and the mixture was heated to 130 °C and stirred for 12 h. The system was cooled to room temperature, water (50 mL) was added, and the mixture was extracted with ethyl acetate (50 mL). The organic phase was separated out, washed with saturated brine (50 mL), dried over anhydrous sodium sulfate, and filtered. The filtrate was concentrated, and the residue was purified by silica gel column chromatography to give the title product **15a**.

### Step 2

### (E)-4-Fluoro-2-isopropyl-5-[2-(quinolin-2-yl)vinyl]benzene-1,3-diol 15

Referring to the synthesis method of step 2 in Example 7, the title product **15** was prepared from intermediate **15a**. LCMS (ESI, m/z): 324.1 [M+H]⁺.

¹H NMR (400 MHz, DMSO-*d₆*, ppm): δ 9.27 (d, *J* = 1.6 Hz, 1H), 9.23 (s, 1H), 8.35 (d, *J* = 8.4 Hz, 1H), 8.00-7.94 (m, 2H), 7.87 (d, *J =* 8.4 Hz, 1H), 7.80-7.73 (m, 2H), 7.58-7.54 (m, 1H), 7.25 (d, *J =* 16.4 Hz, 1H), 6.63 (d, *J* = 6.0 Hz, 1H), 3.48-3.45 (m, 1H), 1.27 (d, *J* = 6.8 Hz, 6H). ¹⁹F NMR (400 MHz, DMSO-*d₆*, ppm): δ -149.88 (1F).

### Example 16

### (E)-4-Fluoro-2-isopropyl-5-[2-(pyridin-2-yl)vinyl]benzene-1,3-diol 16

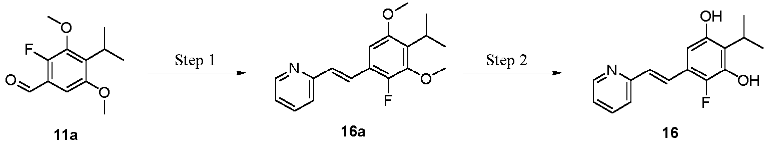

### Step 1

### (E)-2-(2-Fluoro-4-isopropyl-3,5-dimethoxystyryl)pyridine 16a

Referring to the synthesis method of step 6 in Example 1, the title product **16a** was prepared from intermediate **11a** and [(pyridin-2-yl)methyl]triphenylphosphonium bromide.

### Step 2

### (E)-4-Fluoro-2-isopropyl-5-[2-(pyridin-2-yl)vinyl]benzene-1,3-diol 16

Referring to the synthesis method of step 2 in Example 7, the title product **16** was prepared from intermediate **16a**. LCMS (ESI, m/z): 274.0 [M+H]⁺.

¹H NMR (400 MHz, DMSO-*d₆*, ppm): δ 9.20 (s, 1H), 9.15 (s, 1H), 8.58 (d, *J* = 4.4 Hz, 1H), 7.86-7.78 (m, 1H), 7.66 (d, *J =* 16.0 Hz, 1H), 7.62-7.59 (m, 1H), 7.33-7.26 (m, 1H), 7.08 (d, *J* = 16.0 Hz, 1H), 6.56 (d, *J* = 6.0 Hz, 1H), 3.49-3.43 (m, 1H), 1.26 (d, *J* = 7.2 Hz, 6H). ¹⁹F NMR (400 MHz, DMSO-*d₆*, ppm): δ -150.29 (1F).

### Example 17

### (E)-4-Fluoro-2-isopropyl-5-[2-(pyridin-3-yl)vinyl]benzene-1,3-diol 17

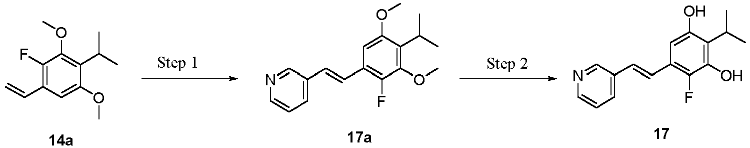

### Step 1

### (E)-3-(2-Fluoro-4-isopropyl-3,5-dimethoxystyryl)pyridine 17a

Referring to the synthesis method of step 2 in Example 14, the title product **17a** was prepared from 3-bromopyridine and intermediate **14a**.

### Step 2

### (E)-4-Fluoro-2-isopropyl-5-[2-(pyridin-3-yl)vinyl]benzene-1,3-diol 17

Referring to the synthesis method of step 2 in Example 7, the title product **17** was prepared from intermediate **17a**. LCMS (ESI, m/z): 274.0 [M+H]⁺.

¹H NMR (400 MHz, DMSO-*d₆*, ppm): δ 9.36-9.05 (m, 2H), 9.00-8.90 (m, 1H), 8.62-8.58 (m, 1H), 8.51-8.38 (m, 1H), 7.75-7.62 (m, 1H), 7.41 (d, *J* = 16.4 Hz, 1H), 7.11 (d, *J* = 16.4 Hz, 1H), 6.54 (d, *J* = 6.0 Hz, 1H), 3.50-3.42 (m, 1H), 1.26 (d, *J =* 7.2 Hz, 6H). ¹⁹F NMR (400 MHz, DMSO-*d₆*, ppm): δ -149.83 (1F).

### Example 18

### (E)-4-Fluoro-2-isopropyl-5-[2-(pyridin-4-yl)vinyl]benzene-1,3-diol 18

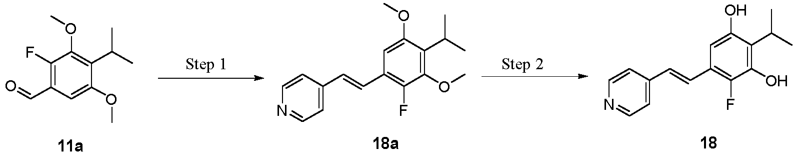

### Step 1

### (E)-4-(2-Fluoro-4-isopropyl-3,5-dimethoxystyryl)pyridine 18a

Referring to the synthesis method of step 1 in Example 15, the title product **18a** was prepared from 4-methylpyridine and intermediate **11a**.

### Step 2

### (E)-4-Fluoro-2-isopropyl-5-[2-(pyridin-4-yl)vinyl]benzene-1,3-diol 18

Referring to the synthesis method of step 2 in Example 7, the title product **18** was prepared from intermediate **18a**. LCMS (ESI, m/z): 274.0 [M+H]⁺.

¹H NMR (400 MHz, DMSO-*d₆*, ppm): δ 9.24 (d, *J* = 2.4 Hz, 1H), 9.19 (s, 1H), 8.56 (d, *J* = 5.6 Hz, 2H), 7.65-7.61 (m, 2H), 7.47 (d, *J =* 16.4 Hz, 1H), 7.02 (d, *J* = 16.4 Hz, 1H), 6.55 (d, *J* = 6.0 Hz, 1H), 3.49-3.43 (m, 1H), 1.26 (d, *J =* 7.2 Hz, 6H). ¹⁹F NMR (400 MHz, DMSO-*d₆*, ppm): δ -149.61 (1F).

### Test Example 1. Luciferase Reporter Gene Assay

In this test example, a luciferase reporter gene assay was performed to assay the agonistic activity effect of the compounds of the present disclosure on AHR protein.

### Test cell:

Human hepatoma cells HepG2-Lucia expressing AHR and luciferase were purchased from InvivoGen with the catalog number of hpgl-ahr;

### Main instruments:

Biosafety cabinet, model: 307, ThermoFisher;
CO₂ incubator, model: CLM-240B-8-CN, ESCO;
Cell counter, model: EVE-MC2, NanoEnTeK;
ECHO (nano-liter scale acoustic liquid handling system), model: 655, LabCyte;
Microplate centrifuge, model: PlatePro 3200, Monad;
Multifunctional microplate reader, model: PHERAstar FSX, BMG LRBTECH.

### Main reagents:

Penicillin-streptomycin, Gibco, Cat. No. 15140-122;
EMEM medium, ATCC, Cat. No. 30-2003;
Fetal bovine serum, Ausgenex, Cat. No. FBS500-S;
NEAA medium, Gibco, Cat. No. 11140-050;
Phosphate buffer, Gibco, Cat. No. 14190250;
DMSO (dimethyl sulfoxide), Solarbio, Cat. No. D8371;
FICZ (6-formylindolo[3,2-B]carbazole), MCE, Cat. No. HY-12451;
Zeocin (bleomycin), InvivoGen, Cat. No. ant-zn-1;
QUANTI-Luc Gold, InvivoGen, Cat. No. rep-qlcg5.

### Experimental procedures:

1. HepG2-Lucia AHR cells were cultured in an EMEM medium containing 10% inactivated fetal bovine serum, 1× NEAA, penicillin-streptomycin, and 100 µg/mL Zeocin (bleomycin). The culture temperature was 37 °C and the concentration of carbon dioxide was 5%.
2. After the cells were grown to about 80% confluency, the cells were digested, centrifuged, resuspended, and counted. The cells were seeded into a 384-well plate at 40 µL/well.
3. Different concentrations of the test compounds were added by using ECHO at 40 nL/well.
4. The 384-well plate to which the compounds were added was incubated in the incubator for 24 h.
5. The supernatant was collected, a QUANTI-Luc Gold detection reagent was added, and the luminescence signal values were read by using the multifunctional microplate reader.

### Test results:

The data for the activity, EC₅₀, of the compounds of the present disclosure and Benvitimod on AHR protein are summarized in Table 1 below:

**Table 1. EC₅₀ for AHR activation by the compounds of the present disclosure**

| Compound | Luciferase-labeled human hepatoma cells (HepG2-Lucia) AHR agonist EC₅₀ (µM) |
|---|---|
| Benvitimod | 0.043 |
| Compound 1 | 0.238 |
| Compound 2 | 0.177 |
| Compound 3 | 0.057 |
| Compound 4 | 0.219 |
| Compound 5 | 0.006 |
| Compound 6 | 0.010 |
| Compound 7 | 0.030 |
| Compound 8 | 0.013 |
| Compound 9 | 0.020 |
| Compound 14 | 0.027 |
| Compound 18 | 0.066 |

The results described above show that the compounds in the examples of the present disclosure had relatively good activation activity on AHR protein, and at least the activities of compounds 5-9 and 14 were remarkably superior to that of Benvitimod.

### Test Example 2. Illumination Stability Experiment

In this test example, the stability of the compounds in the examples of the present disclosure, Benvitimod, and the compound of Comparative Example 1 under illumination conditions was tested and compared.

### Main instruments:

Stability chamber, model: ICH-110L, Memmert, USA;
High performance liquid chromatograph (HPLC), model: 1260, Agilent.

### Analysis method:

Detection wavelength: 220 nM, 254 nM
Chromatographic column: Agilent ZORBAX SB-C8 4.6 × 250 mm, 5 µm
Mobile phase A: water
Mobile phase B: acetonitrile
Flow rate: 1.0 mL/min
Column temperature: 35 °C
Injection volume: 10 µL

### Experimental procedures:

1. A test compound was weighed out and placed into the stability chamber;
2. a light source was turned on, with visible light of 5000±500 lx and ultraviolet light of 250 µW/cm²;
3. sampling was performed at 0 h, 8 h, 24 h, and 72 h, and the sample was dissolved in an aqueous acetonitrile solution with a volume fraction of 50%;
4. HPLC was used for detection, and the content was determined by using the area normalization method.

### Test results:

The stability data of the compounds of the present disclosure and Benvitimod in different batches under the illumination conditions are summarized as follows:

**Table 2. Stability of the compound of the present disclosure under the illumination conditions (batch 1)**

| Compound | Light source condition | Liquid chromatography content (220 nM, %) | | | |
|---|---|---|---|---|---|
| | | 0h | 8h | 24h | 72h |
| Benvitimod | Visible light 5000±500 lx; ultraviolet light 250 µW/cm² | 99.87% | 85.14% | 74.20% | 69.53% |
| Compound 3 | | 97.25% | 97.33% | 96.06% | 97.71% |

**Table 3. Stability of other compounds of the present disclosure under the illumination conditions (batch 2)**

| Compound | Structure | | Liquid chromatography content (220 nM, %) | | | |
|---|---|---|---|---|---|---|
| | | | 0h | 8h | 24h | 72h |
| Compound 5 | 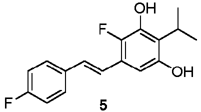 | Light source condition Visible light 5000±500 lx; ultraviolet light 250 µW/cm² | 99.30 | 98.80 | 99.22 | 99.06 |
| Compound 6 | 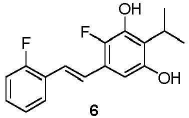 | | 98.31 | 98.37 | 98.27 | 98.68 |
| Compound 7 | 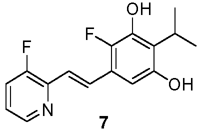 | | 98.70 | 95.84 | 91.56 | 89.57 |
| Compound 9 | 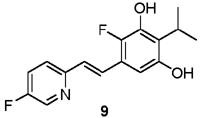 | | 99.46 | 98.65 | 97.46 | 92.06 |
| Compound 14 | 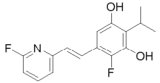 | | 88.32 | 87.83 | 88.31 | 84.37 |
| Comparative Example 1 | 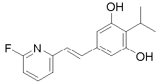 | | 96.99 | 85.98 | 82.13 | 72.30 |
| Compound 16 | 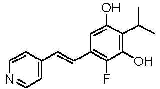 | | 97.58 | 97.52 | 96.88 | 96.81 |

Among them, the compound of Comparative Example 1 was a compound synthesized by the inventors.

The results described above show that the compounds in the examples of the present disclosure were more stable than Benvitimod and the compound of Comparative Example 1 under the illumination conditions: the compound contents and/or the degree of changes in the content of the compounds in the examples of the present disclosure at 8 h, 24 h, and 72 h were remarkably superior to those of Benvitimod and/or the compound of Comparative Example 1, indicating that the photostability of the compounds of the present disclosure is significantly improved.

## Claims

1. A compound represented by formula I-1 and a stereoisomer, a pharmaceutically acceptable salt or a prodrug thereof: 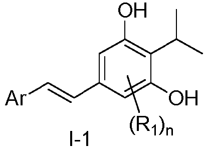
wherein Ar is selected from the following groups unsubstituted or optionally substituted with one, two, or more Rs: C₆₋₂₀ aryl and 5- to 20-membered heteroaryl;
each Rs is identical or different and is independently selected from halogen, cyano, C₁₋₁₂ alkyl, halogenated C₁₋₁₂ alkyl, -COC₁₋₁₂ alkyl, and C₁₋₁₂ alkoxy;
each R₁ is identical or different and is independently selected from halogen, cyano, C₁₋₁₂ alkyl, halogenated C₁₋₁₂ alkyl, -COC₁₋₁₂ alkyl, and C₁₋₁₂ alkoxy; and
n is 1 or 2.

2. The compound represented by formula I-1 and the stereoisomer, the pharmaceutically acceptable salt or the prodrug thereof according to claim 1, wherein each R₁ is identical or different and is independently selected from halogen, cyano, C₂₋₆ linear alkyl, halogenated C₁₋₆ alkyl, -COC₂₋₆ alkyl, and C₁₋₆ alkoxy.

3. The compound represented by formula I-1 and the stereoisomer, the pharmaceutically acceptable salt or the prodrug thereof according to claim 1 or 2, being a compound represented by formula I below and a stereoisomer, a pharmaceutically acceptable salt or a prodrug thereof: 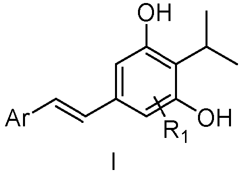 wherein:
Ar is selected from the following groups unsubstituted or optionally substituted with one, two, or more Rs: C₆₋₂₀ aryl and 5- to 20-membered heteroaryl;
each Rs is identical or different and is independently selected from halogen, C₁₋₁₂ alkyl, halogenated C₁₋₁₂ alkyl, - COC₁₋₁₂ alkyl, and C₁₋₁₂ alkoxy;
R₁ is as defined in claim 1.

4. The compound represented by formula I-1 and the stereoisomer, the pharmaceutically acceptable salt or the prodrug thereof according to any one of claims 1 to 3, wherein Ar is selected from the following groups unsubstituted or optionally substituted with one, two, or more Rs: phenyl and 5- to 6-membered heteroaryl; Rs is as defined in claim 1; preferably, Ar is selected from the following groups unsubstituted or optionally substituted with one, two, or more Rs: phenyl, pyridin-2-yl, pyridin-3-yl, pyridin-4-yl, thienyl, quinolyl, isoquinolyl, pyridazinyl, pyrazinyl, and pyrimidinyl; Rs is as defined in claim 1.

5. The compound represented by formula I-1 and the stereoisomer, the pharmaceutically acceptable salt or the prodrug thereof according to any one of claims 1 to 4, wherein each R₁ is identical or different and is independently F, Cl, Br, methyl, or cyano.

6. The compound represented by formula I-1 and the stereoisomer the pharmaceutically acceptable salt or the prodrug thereof according to any one of claims 1 to 5, wherein Ar is selected from the following groups unsubstituted or optionally substituted with one, two, or more Rs: phenyl, pyridin-2-yl, pyridin-3-yl, pyridin-4-yl, 2-thienyl, 2-pyridazinyl, and 2-quinolyl; Rs is as defined in claim 1;
R₁ is selected from F, Cl, Br, cyano, C₁₋₃ alkyl, halogenated C₁₋₃ alkyl, -COC₁₋₃ alkyl, and C₁₋₃ alkoxy, preferably F, Cl, Br, cyano, C₁₋₃ alkyl, or halogenated C₁₋₃ alkyl.

7. The compound represented by formula I-1 and the stereoisomer, the pharmaceutically acceptable salt or the prodrug thereof according to any one of claims 1 to 6, wherein Ar is selected from 4-fluorophenyl, 2-fluorophenyl, 2-pyridazinyl, 2-thienyl, 2-quinolyl, 2-pyridinyl, 3-pyridinyl, 4-pyridinyl, 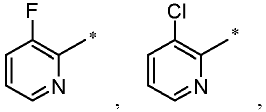 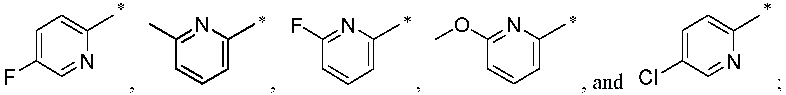 R₁ is selected from F, Cl, Br, methyl, methoxy, cyano, acetyl, 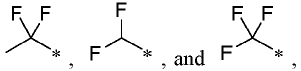 preferably F, Cl, Br, cyano,

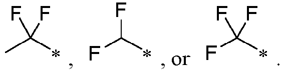

8. The compound represented by formula I-1 and the stereoisomer, the pharmaceutically acceptable salt or the prodrug thereof according to any one of claims 1 to 7, wherein each Rs is identical or different and is independently selected from halogen, C₁₋₆ alkyl, and C₁₋₆ alkoxy.

9. The compound represented by formula I-1 and the stereoisomer, the pharmaceutically acceptable salt or the prodrug thereof according to any one of claims 1 to 8, wherein the compound represented by formula I-1 is selected from the following compounds: 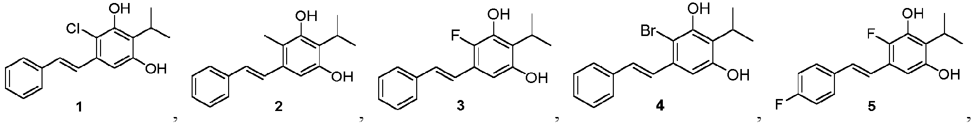 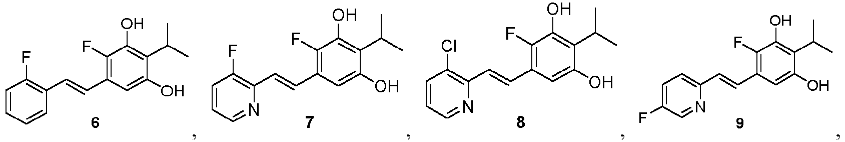 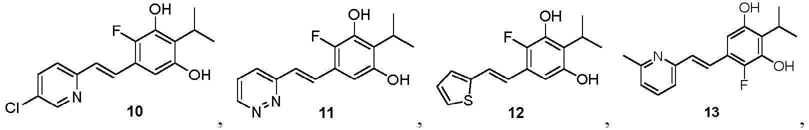 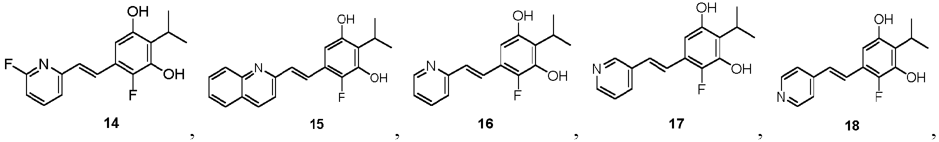 and

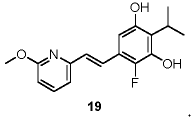

10. A compound represented by general formula I-1d or a salt thereof: 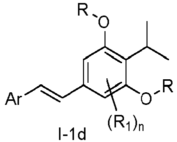 wherein:
R is selected from alkyl, hydroxyalkyl, cycloalkyl, heterocyclyl, aryl, and heteroaryl, wherein the alkyl, cycloalkyl, heterocyclyl, aryl, and heteroaryl are each independently and optionally substituted with one or more substituents selected from the following groups: halogen, oxo, alkyl, haloalkyl, alkoxy, haloalkoxy, nitro, cyano, amino, alkylamino, hydroxyl, hydroxyalkyl, cycloalkyl, heterocyclyl, aryl, and heteroaryl; preferably, R is C₁₋₆ alkyl;
each R₁ is identical or different and is independently selected from halogen, cyano, C₁₋₁₂ alkyl, halogenated C₁₋₁₂ alkyl, -COC₁₋₁₂ alkyl, and C₂₋₁₂ alkoxy, preferably F, Cl, Br, cyano, C₁₋₃ alkyl, or halogenated C₁₋₃ alkyl;
Ar and n are as defined in claim 1.

11. The compound represented by general formula I-1d or the salt thereof according to claim 10, wherein the compound is selected from the following compounds: 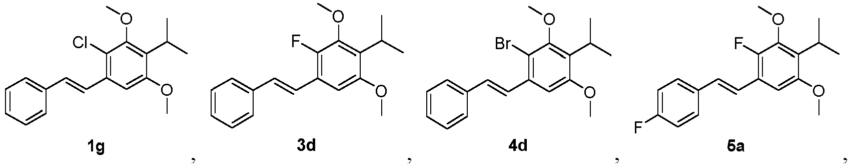 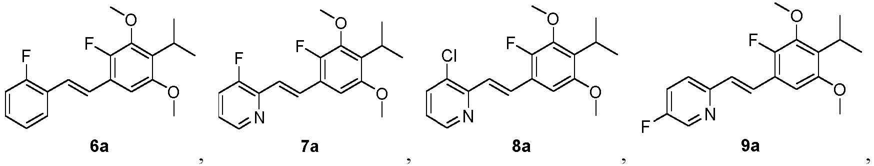 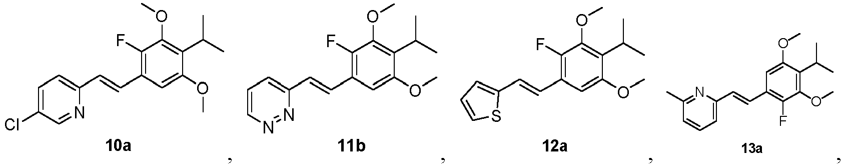 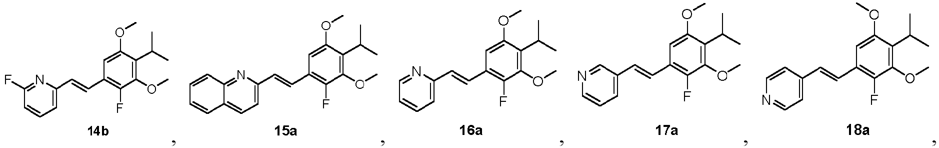 and

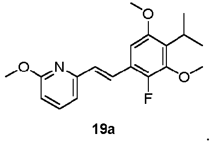

12. A preparation method for the compound represented by formula I and the stereoisomer or the pharmaceutically acceptable salt thereof according to claim 3, comprising the following steps:
1) when R₁ is halogen, the compound represented by formula I is prepared by the following method: 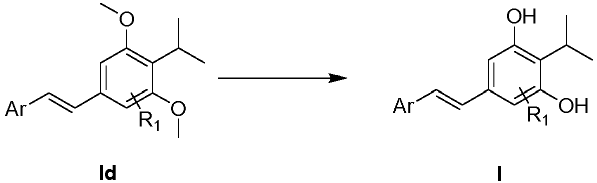 S5) subjecting a compound represented by formula Id or a salt thereof and pyridine hydrochloride to a heating reaction to give the compound represented by formula I and the stereoisomer or the pharmaceutically acceptable salt thereof; or removing methyl from the compound represented by formula Id or the salt thereof by using boron tribromide, and then quenching the reaction with water to give the compound represented by formula I and the stereoisomer or the pharmaceutically acceptable salt thereof;
2) when R₁ is methyl, the compound represented by formula I is prepared by the following method: 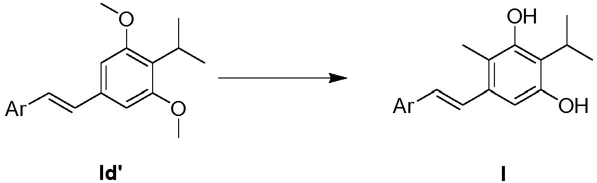
S5') subjecting compound Id' and pyridine hydrochloride to a heating reaction to give the compound represented by formula I;
Ar is as defined in claim 1.

13. A pharmaceutical composition, comprising a therapeutically effective amount of at least one of the compound represented by formula I-1 and the stereoisomer, the pharmaceutically acceptable salt or the prodrug thereof according to any one of claims 1 to 9.

14. The pharmaceutical composition according to claim 13, further comprising one or more pharmaceutically acceptable carriers or excipients.

15. The pharmaceutical composition according to claim 13 or 14, wherein the pharmaceutical composition is an aryl hydrocarbon receptor (AHR) regulator.

16. Use of at least one of the compound represented by formula I-1 and the stereoisomer, the pharmaceutically acceptable salt or the prodrug thereof according to any one of claims 1 to 9, or the pharmaceutical composition according to any one of claims 13 to 15 for manufacturing an aryl hydrocarbon receptor (AHR) regulator, wherein the aryl hydrocarbon receptor (AHR) regulator is used for the alleviation and/or treatment of the following diseases or conditions: a cancer, an ophthalmology-related disease, an autoimmune disease, and other conditions or discomfort with an immunological factor; the cancer is preferably leukemia, prostate cancer, and intestinal cancer; the ophthalmology-related disease is preferably uveitis, age-related macular degeneration, and dry eye syndrome; the autoimmune disease is preferably rheumatoid arthritis, multiple sclerosis, systemic lupus erythematosus, inflammatory bowel disease, type 1 diabetes, vitiligo, atopic dermatitis, and psoriasis; said other conditions or discomfort with the immunological factor are preferably asthma, allergy, infection, osteoporosis, atherosclerosis, type 2 diabetes, graft-versus-host disease, and graft rejection.
